# FASCICULE DE BREVET EUROPEEN

(11) **EP 0 445 128 B1**
(45) Date de publication et mention de la délivrance du brevet: **07.06.1995**
(21) Numéro de dépôt: 89911564.6
(22) Date de dépôt: 27.09.1989
(51) Int. Cl.: C12N 15/62, C07K 2/00, C12N 15/31, C12N 15/40, C12N 15/43, C12N 15/50, A61K 39/106, A61K 39/13, A61K 39/225, A61K 39/295, A61K 39/39

(54) **PROTEINES DE FUSION DE LA SOUS-UNITE B DE LA TOXINE CHOLERIQUE ET D'UN ANTIGENE HETEROLOGUE ET ACIDES NUCLEIQUES LES ENCODANT**
FUSIONSPROTEINE DER UNTEREINHEIT B VON CHOLERATOXIN UND EINES HETEROLOGEN ANTIGENS UND DAFÜR KODIERENDE REKOMBINANTE NUKLEINSÄURE
PROTEINS FOR FUSING THE SUB-UNIT B OF THE CHOLERAIC TOXIN AND HETEROLOGOUS ANTIGEN, AND NUCLEIC ACIDS ENCODING THEM

(30) Priorité: 27.09.1988 FR 8812627
(43) Date de publication de la demande: 11.09.1991
(73) Titulaire: L'Hoir, Cécile, B-4020 Liège (BE); Renard, André, B-4031 Liège (BE); Martial, Joseph, B-4130 Esneux (BE)
(72) Inventeur: L'Hoir, Cécile, B-4020 Liège (BE); Renard, André, B-4031 Liège (BE); Martial, Joseph, B-4130 Esneux (BE)
(74) Mandataire: Desaix, Anne
(86) Numéro de dépôt international: FR8900495
(87) Numéro de publication internationale: WO9003437

(56) Documents cités:
- EP-A- 0 095 452
- WO-A-86/06635
- The Journal of Immunology, vol. 133, no. 4, octobre 1984, The American Association of Immunologists, (US), S.J. McKenzie et al.; pp. 1818-1824
- J. Gen. Virol., vol. 68, 1987, SGM, (GB), D. Rasschaert et al.; pp. 1883-1890
- Nucleic Acids Research, vol. 11, no. 12, Juin 1983, IRL-Press Ltd. (Oxford, GB), M. L. Gennaro et al.; pp. 3855-3861
- Nature, vol. 306, no. 5943, 8 decembre 1983, Macmillan Journals Ltd, (Chesham, Bucks, GB), J.J. Mekalanos et al.; pp. 551-557
- Gene, vol. 55, nos. 2-3, 1987, Elsevier Science Publishers B.V. (Biomedical Division), (Amsterdam, NL), A. Phalipon et al.; pp. 255-263
- The Journal of Immunology, vol. 141, no. 5, 1er septembre 1988, The American Association of Immunologists, (US), X. Liang et al.; pp. 1495-1501
- FEBS Letters, vol. 241, nos. 1, 2, decembre 1988, Elsevier Science Publishers B.V. (Biomedical Division), J. Sanchez et al.; pp. 110-114

## Description

L'invention concerne une protéine de fusion dérivée de la sous-unité B de la toxine cholérique (ci-après désignée sous l'abréviation CTB) et fusionnée avec une séquence active d'une séquence polypeptidique hétérologue vis-à-vis de ladite sous-unité B et témoignant d'une activité Iga immunogénique, ces protéines hybrides conservant la spécificité d'attachement de la CTB aux gangliosides GM1 et possédant simultanément l'activité Iga antigénique de la protéine hétérologue.

Il est entendu que, dans ce qui suit, l'expression "protéine" recouvre aussi bien une protéine active, qu'un polypeptide ayant seulement l'ossature polypeptidique de ladite protéine active, en particulier lorsque celle-ci comprend d'autres groupes chimiques, par exemple des groupes de glycosylation et vice-versa.

La protéine hybride selon l'invention est caractérisée en ce qu'elle résulte de la fusion directement ou par l'intermédiaire d'éventuels résidus aminoacyle de liaison d'une séquence issue de la CTB et d'une séquence antigénique hétérologue vis-à-vis de la CTB, cette fusion étant telle que soit essentiellement conservée la configuration pentamérique que la CTB forme à l'état naturel, et que par ailleurs soient conservées à la fois dans la protéine de fusion la spécificité d'attachement de la CTB au ganglioside GM1 et l'activité Iga antigénique de la protéine hétérologue.

Une protéine hybride ou de fusion préférée est caractérisée par le fait qu'elle comporte une séquence de ladite sous-unité s'étendant entre le 3ème et le 100ème résidus d'acides aminés de la sous-unité B de la CTB et comptés à partir de l'extrémité N-terminale de cette sous-unité et que la séquence issue de la sous-unité B est fusionnée en amont ou en aval de celle-ci avec la séquence active de l'antigène hétérologue.

De préférence, la fusion est réalisée génétiquement comme il sera décrit plus loin. Sans que les indications chiffrées qui suivent aient un caractère limitatif, il peut être indiqué que de préférence la protéine hétérologue fusionnée avec la séquence issue de la CTB comprend notamment de 4 à 100 et de préférence 5 à 50 résidus d'acides aminés. Cette protéine hétérologue comprend plus particulièrement, notamment lorsqu'elle est destinée à la production de vaccins, les épitopes plus particulièrement responsables de l'activité neutralisante de ladite protéine à l'égard de l'agent pathogène correspondant vis-à-vis de l'hôte.

A titre d'arrière-plan technique de l'invention, on rappelle que la production d'IgAs secrétoires joue un rôle important dans la neutralisation des virus présents dans la lumière intestinale.

Cette réponse immunitaire spécifique requiert la stimulation préalable, par l'antigène, des tissus lymphoïdes associés à l'intestin (plaques de Peyer, ganglions lymphatiques mésentériques). Cependant, beaucoup d'antigènes (Ags) ne sont pas "IgA-géniques", vraisemblablement parce qu'ils ne peuvent pas rester en contact prolongé avec la paroi de la muqueuse intestinale (Bulletin de l'O.M.S., 1980).

Les stratégies générales de vaccination utilisées pour la prévention d'infections systémiques ne sont généralement pas applicables contre les virus qui ont pour cible la surface de la muqueuse intestinale (Bulletin de l'O.M.S, 1980). Le contrôle prophylactique des infections entériques implique donc des stratégies nouvelles spécialement adaptées.

Dans cette invention, nous avons développé une méthode générale pour stimuler la réponse immunitaire au niveau de l'intestin. Le principe de base sur lequel cette invention est basée consiste à augmenter l'activité antigénique de l'antigène au niveau de l'interaction avec les tissus lymphoïdes de l'intestin. Notre méthode repose sur le couplage de deux principes :
(1) l'augmentation du contact avec l'intestin à l'aide d'un adjuvant qui est couplé à l'antigène et
(2) la stabilisation de l'antigène dans l'environnement internal, afin de prolonger la survie de l'antigène.

Dans un mode de mise en oeuvre préféré de l'invention, le couplage d'une façon stable de l'antigène à la CTB est réalisé par génie génétique entre des fragments d'acides nucléiques codant respectivement pour les deux constituants protéiques, pour créer un couplage protéique entre l'antigène "IgA-génique" et la séquence issue de la CTB.

La sous-unité B de la toxine cholérique (CTB) est une protéine multimérique (PM 85.000) s'attachant aux cellules épithéliales de l'intestin. Elle est composée de deux sous-unités : une sous-unité A (PM 27.000) responsable de l'activité biologique et 5 sous-unités B (PM 11.600) organisées en pentamère qui se fixent sur les récepteurs cellulaires, les gangliosides GM1 (Holmgren et al., 1975). La toxine entière, ainsi que sa sous-unité B seule (CTB), sont des immunogènes forts de la muqueuse intestinale (Pierce, 1978 ; McKenzie et Halsey, 1983 ; Elson et Ealdin, 1984 ; Lycke et Holmgren, 1986 et 1987).

Afin de contrôler plus efficacement la spécificité du couplage et d'optimaliser la fusion, nous proposons de coupler les antigènes à la protéine CTB par la voie génétique. Pour cela nous avons utilisé la technique de fusion de gènes, qui permet d'obtenir des protéines couplées ou fusionnées. Bien que la fusion de gènes comme technique pour obtenir des protéines couplées est une méthode générale, le résultat du couplage ne peut toujours être prédit avec certitude. Dans la majorité des cas, on observe que la protéine de fusion est instable et est très vite dégradée avec la synthèse. Encore moins évident est la production de protéines couplées qui retiennent les activités enzymatiques ou biologiques des deux protéines originales. En effet, on n'obtient que très rarement des protéines couplées qui possèdent les activités biologiques des deux protéines. Ceci est dû au fait que le processus de couplage lui-même a une influence importante sur la façon dont les composantes polypeptidiques de fusion se replient, ayant pour conséquence que la conformation tridimensionnelle du polypeptide est différente de la configuration de la protéine native. L'activité biologique dépend strictement de la conformation tridimensionnelle exacte. En conséquence, on observe que l'activité biologique se perd souvent dans des protéines de fusion.

Dans cette invention, nous avons réussi à obtenir des protéines de fusion qui combinent les activités biologiques des deux composants polypeptidiques, c'est-à-dire la spécificité d'attachement aux gangliosides GM1 de la CTB, l'effet adjuvant de la CTB et l'activité Iga antigénique issue de la protéine antigène. L'invention repose sur la découverte que l'activité d'attachement spécifique de la CTB et la structure tridimensionnelle de la CTB pouvaient être conservées, notamment lorsque la protéine hétérologue est fusionnée à l'une ou à l'autre des deux extrémités de la protéine CTB monomère (ou aux deux à la fois). Ces extrémités forment par conséquent des sites préférés d'attachement des protéines antigènes. L'invention porte aussi sur le développement d'un système de couplage qui permettrait de coupler n'importe quel antigène à l'adjuvant CTB. Ceci a été réalisé avec la construction de vecteurs d'expression où le couplage est indépendant de l'Ag considéré et qui pourrait être utilisé pour une batterie d'Ags (vecteur "cassette").

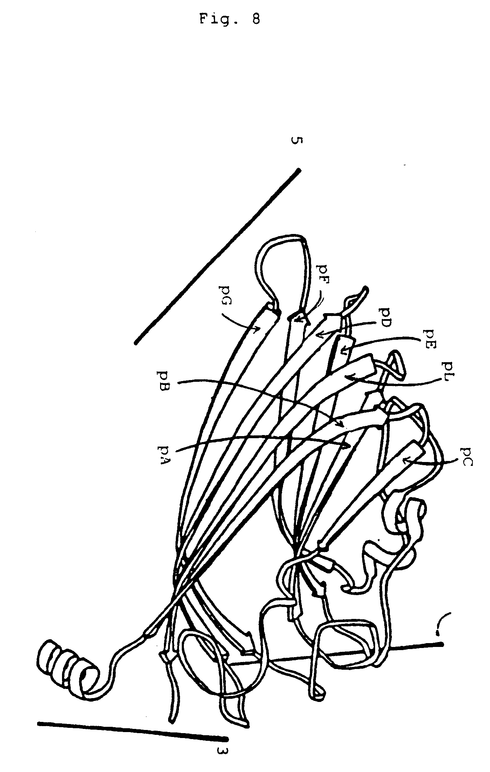

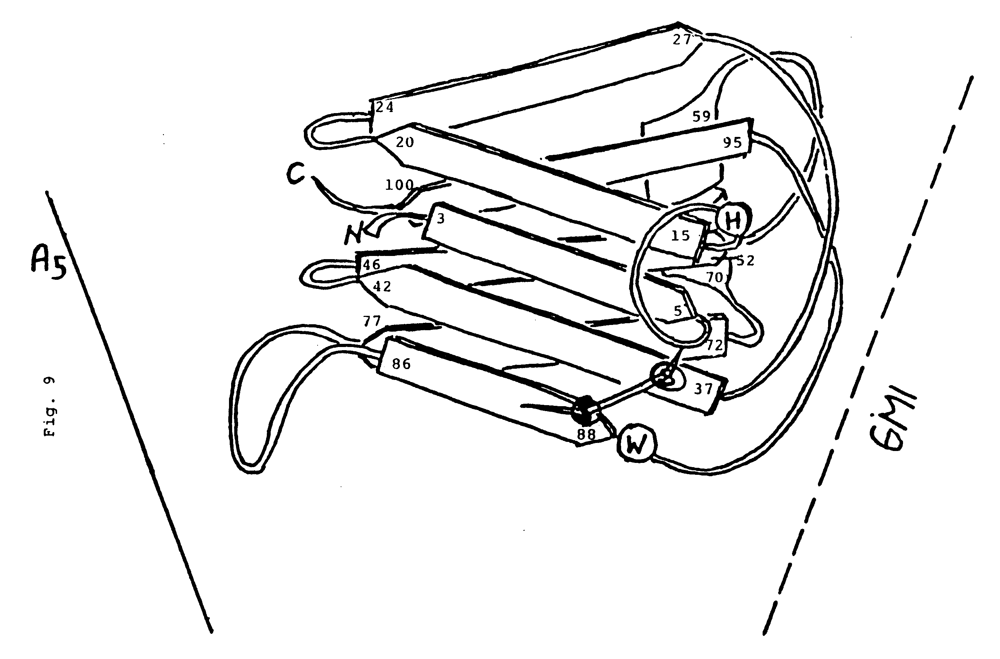

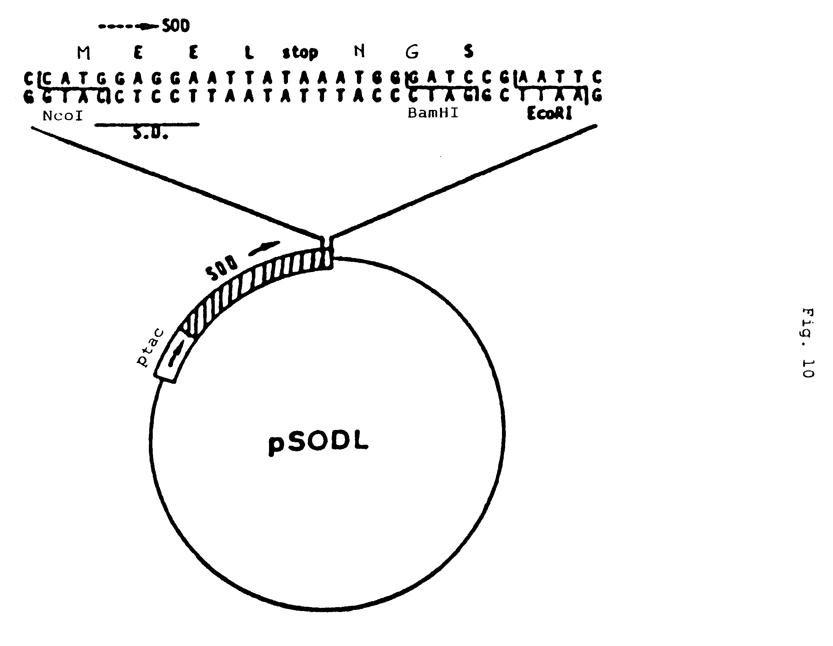

Ces résultats sont d'autant plus remarquables que la structure tridimensionnelle de la protéine CTB est très complexe : (1) Fishman et al. (1978) ont démontré que la protéine du type "allβ " contenait essentielle-ment des régions avec une configuration bêta (β-sheet) et (2) la CTB forme des pentamères avec un seul axe de symétrie d'ordre 5 (Divyer et Bloomfeld, 1982). Nous avons utilisé les algorithmes de prédiction de structure secondaire de Chou et Fasman (1978), de Cohen et al. (1986) et de Garnier et al. (1982) pour identifier les régions de structure secondaire dans la séquence d'acides aminés de la CTB. Nous avons retrouvé 8 régions distinctes de configuration bêta séparées par des régions en configuration de boucle. Ce type d'organisation d'éléments de structures secondaires est typique de la famille des protéines dénommées "tonneau bêta", où le noyau central de la protéine est formé par 8 chaînes en configuration β antiparallèles. En effet, il existe un ensemble de protéines dont la structure a été déterminée par analyse aux rayons X ayant exactement les mêmes caractéristiques : ce sont les protéines de la capside (Rossman et al., 1983) et les picornavirus (Hoghe et al., 1985 ; Rossman et al., 1985). Le coeur de ces protéines, fort similaire malgré l'absence d'homologie de séquences, est constitué par un tonneau de 8 brins β-antiparallèles en forme de "tranche de tarte", la pointe étant orientée vers l'axe d'ordre 5 (figure 8). Ces protéines sont toutes plus longues (150 à 300 acides aminés) que la sous-unité B (103 acides aminés). Dans ces protéines, une grande partie de la structure des boucles externes opposées à l'axe d'ordre 5 et une partie du tonneau central, sont responsables des zones de contact sur les axes d'ordre 3 et d'ordre 2 et assurent la stabilité de la capside virale, et sont donc spécifiques de ces protéines. D'autre part, une série d'autres protéines avec un tonneau β-antiparallèle, comme les différents domaines des immunoglobines, ont une taille comparable à CTB. Nous avons utilisé les règles d'empaquetage de Ptitsyn (1981) pour prédire la topologie du tonneau et le résultat de notre prédiction est représenté dans la figure 9 et que nous pouvons décrire comme suit : (1) Les 8 brins bêta antiparallèles contiennent les résidus 3 à 5 ; 15 à 20 ; 24 à 27 ; 37 à 42 ; 46 à 52 ; 72 à 77 ; 86 à 88 et 95 à 100. (2) Des grandes boucles externes, exposées au solvant contiennent les résidus 6 à 14; 28 à 36 ; 53 à 71 et 89 à 94. (3) Les petites boucles internes de l'axe de symétrie contiennent les résidus 21 à 23 ; 43 à 45 et 78 à 85. (4) Les deux extrémités NH₂ et COOH se trouvent au centre de l'axe de symétrie et ne contiennent que quelques résidus : 2 à l'extrémité NH₂ est 3 à l'extrémité COOH. (5) Une hélice α comprenant les résidus 59-70 située dans la grande boucle des résidus 53 à 71.

Cette structure tridimensionnelle est en accord avec plusieurs observations antérieures :
1) Les deux cystéines sont suffisamment proches pour former le pont disulfure (figure 9).
(2) Les acides aminés impliqués dans la liaison avec GM1 (Dewolf et al., 1981) sont sur la même face de la protéine accessible au solvant. De même, les peptides antigéniques (Sela et al., 1984) sont sur cette face exposée au solvant (figure 9).
(3) Les peptides non antigéniques sont soit dans le tonneau, soit sur les boucles de l'axe de symétrie.

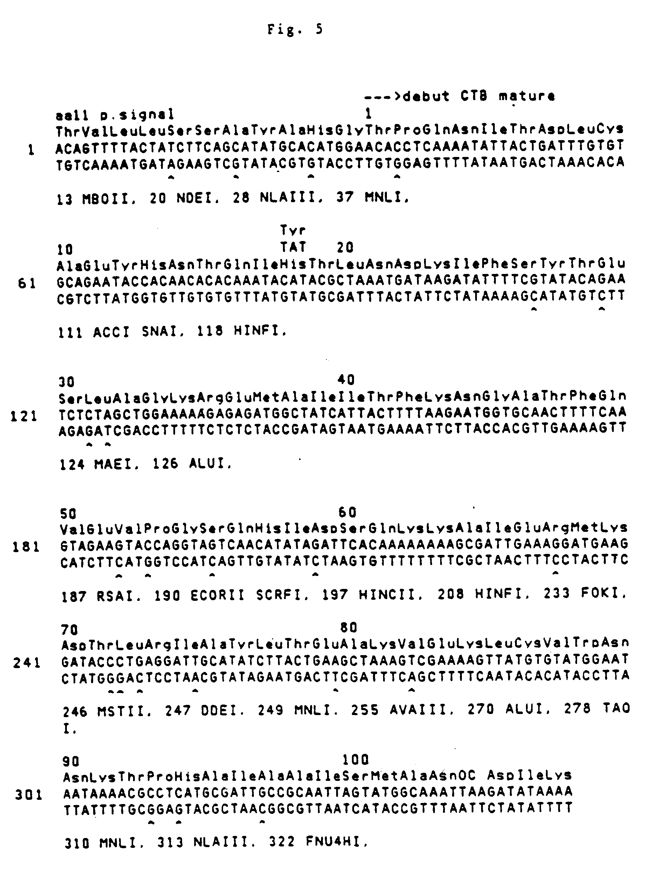

Finalement, l'organisation des brins "externes" et "internes" du tonneau est en accord avec la prédiction à l'aide du moment hydrophobique (Eisenberg et al., 1984). C'est dire qu'il n'était pas évident que la CTB pouvait être utilisée comme protéine porteuse d'antigènes étrangers, sans que soient pour autant affectées ses propriétés d'attachement au récepteur cellulaire GM1 et tout en conservant les facultés de la protéine antigénique hétérologue à être exposée de façon à pouvoir être reconnue par des anticorps spécifiques à cette protéine antigénique notamment par des anticorps neutralisant l'agent pathogène dont l'antigène peut être issu. Comme l'attachement du récepteur cellulaire est probablement effectué par les boucles externes exposées au solvant, il en résulte que l'insertion d'un épitope d'antigène étranger dans une de ces boucles ne doit pas causer une interférence avec l'attachement au ganglioside GM1, soit directement en détruisant le site d'attachement, soit indirectement en causant un empêchement stérique. La fusion ou l'insertion ne doit pas être effectuée dans les boucles de l'axe de symétrie pour empêcher la formation du pentamètre. L'insertion dans les brins bêta ne doit pas davantage déstabiliser la structure du tonneau. Des endroits préférés pour attacher des chaînes d'acides aminés sans perturber la structure monomérique ou pentamérique et sans interférer avec son attachement au récepteur, sont dans le voisinage de l'une ou l'autre des deux extrémités NH₂ et COOH (figure 9). De plus, comme les résidus 3 et 100 font partie de la structure centrale du tonneau, l'attachement de l'antigène étranger se fait de préférence au-delà de ces deux résidus si l'on veut préserver la structure tridimensionnelle. Il est avantageux de laisser les deux premiers résidus NH₂ terminaux et les trois résidus COOH terminaux inchangés lors de l'attachement de l'épitope antigène étranger. En particulier, le couplage peut se faire respectivement aux résidus 2, 3, 100, 101 et 102 de la protéine mature (fig. 5).

L'invention concerne aussi le procédé de production d'un antigène hybride conforme à l'invention. Il comprend notamment :
- la culture d'un hôte cellulaire compétent auparavant transformé avec un acide nucléique contenant une séquence de nucléotides codant pour la susdite protéine hybride, elle-même placée sous le contrôle d'éléments de régulation, dont notamment un promoteur reconnu par les polymérases de cet hôte cellulaire compétent, les éléments d'initiation et de terminaison de la traduction, et
- la récupération de la protéine hybride produite à partir des produits d'expression de cet hôte cellulaire.

Des séquences de nucléotides préférées, à mettre en oeuvre dans le procédé selon l'invention seront indiquées plus loin.

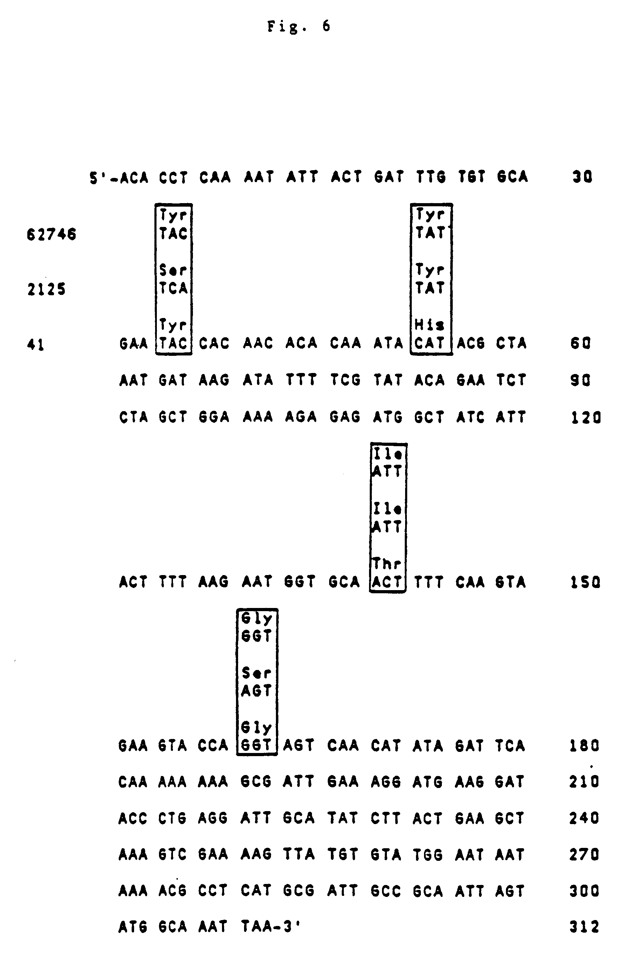

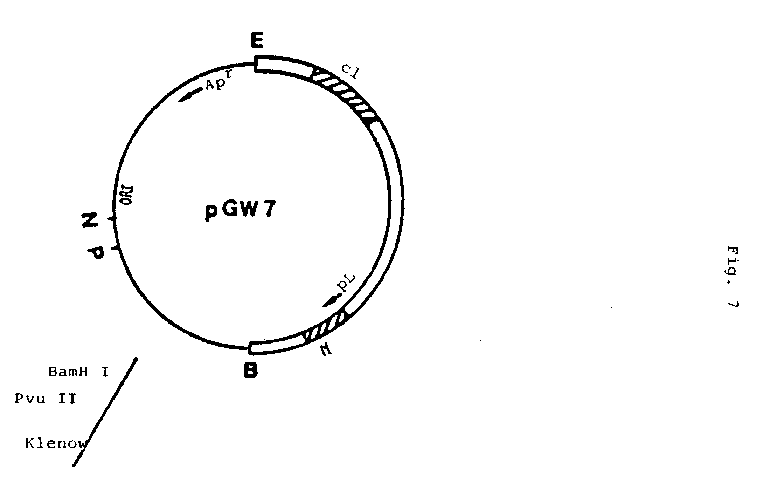

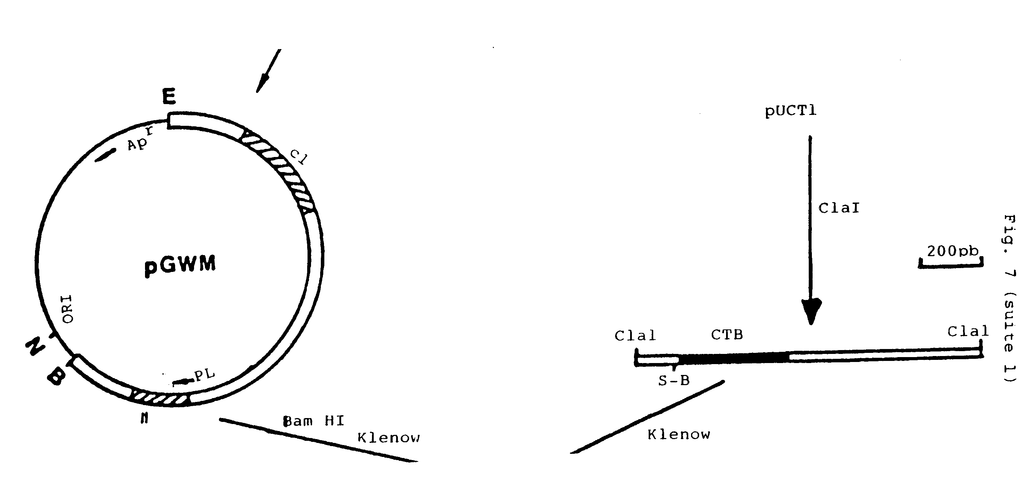

L'invention concerne aussi les protéines hybrides dont les séquences se distinguent des précédentes par un ou plusieurs acides aminés, sans pour autant perdre les capacités à la fois à s'attacher aux gangliosides GM1 et à former des complexes antigène/anticorps avec des anticorps neutralisant la séquence issue de la protéine et, le cas échéant, à induire in vivo des anticorps neutralisants contre l'agent pathogène dont l'antigène hétérologue est issu. De ce point de vue, l'attention doit être attirée sur les figures 5 et 6 dans lesquelles de telles modifications apparaissent à titre d'exemples.

L'acide nucléique recombinant utilisé, codant pour la protéine hybride, est le cas échéant associé à une séquence signal appropriée, le cas échéant hétérologue vis-à-vis de la CTB, chaque fois que le système vecteur-hôte cellulaire mis en oeuvre pour l'expression de la séquence codant pour la protéine de l'invention (sous réserve que le peptide signal ait été convenablement choisi) met à profit la capacité de l'hôte cellulaire, procaryote ou eucaryote (en particulier *E*.coli, levure ou cellule de mammifère) à exploiter l'information susceptible de lui être fournie par la séquence d'ADN signal pour assurer le transport de la protéine hybride vers le périplasme, voire son excrétion dans le milieu de culture de l'hôte cellulaire, après clivage du peptide signal.

Avantageusement, le fragment d'acide nucléique codant pour la séquence 3-100 de la partie CTB de la protéine hybride résulte des figs. 5 et 6. Il peut naturellement s'en différencier par toutes modifications autorisées par la dégénérescence du code génétique ou même davantage, sous réserve que ces modifications (tenant par exemple à des mutations) n'entraînent pas la disparition des propriétés rappelées plus haut de la protéine hybride.

Appartiennent également à l'invention, les acides nucléiques décrits ci-dessus, précédés d'une séquence nucléotidique comprenant les éléments de régulation y inclus le promoteur sous le contrôle duquel s'effectue la transcription de la séquence d'ADN recombinant, les éléments initiateurs et terminateurs de la traduction qui lui sont normalement associés aux gènes dérivés de l'hôte cellulaire.

L'invention concerne aussi les vecteurs recombinants de ce type, phagemides, cosmides, plasmides, phages modifiés par un acide nucléique codant pour la protéine hybride en l'un de leurs sites non essentiels pour leur réplication.

Dans un mode de réalisation particulier de l'invention, le vecteur recombinant ci-dessus est un vecteur d'expression contenant, en l'un de ses sites non essentiels pour se réplication, des éléments nécessaires pour promouvoir l'expression, dans un hôte cellulaire, d'un acide nucléique codant pour la protéine hybride de l'invention, un promoteur compatible avec ledit hôte cellulaire, en particulier un promoteur inductible, et éventuellement une séquence signal.

L'invention se rapporte encore aux hôtes cellulaires transformés par un vecteur recombinant tel que décrit plus haut, vecteur capable de se répliquer dans ledit micro-organisme et comprenant les éléments de régulation permettant l'expression de la séquence nucléique codant pour la protéine hybride de l'invention dans cet hôte.

Un premier hôte cellulaire préféré est constitué par E.coli transformé par un vecteur recombinant tel qu'il sera décrit dans les exemples qui suivent.

L'invention concerne encore plus particulièrement des cultures cellulaires transformées dans les conditions sus-indiquées et aptes à synthétiser la protéine hybride selon l'invention, ces cultures cellulaires étant choisies parmi celles qui peuvent être utilisées pour recoloniser l'intestin de l'hôte animal ou humain auquel le vaccin selon l'invention est destiné. En particulier, on mentionnera les entérobacteriaceae, parmi lesquelles par exemple des souches de E.coli non pathogènes ou des souches atténuées ou virulentes de Salmonella typhi.

L'invention concerne également d'une façon générale les compositions de vaccins comprenant des doses efficaces des protéines hybrides selon l'invention ou des cultures cellulaires aptes à les produire et susceptibles d'être administrées à l'homme ou à l'animal.

Sans qu'il y ait lieu de s'y limiter, on mentionnera comme antigène hétérologue qu'il est avantageux de fusionner avec la CTB un antigène dérivé de la protéine du virus gastroentérique du porc (pour l'immunisation contre la maladie causée chez le même animal par ce virus) ou une protéine de la diarrhée bovine virale (également pour l'immunisation contre la maladie causée par ce virus).

Bien que la fusion génétique représente le mode de production préféré des protéines recombinantes selon l'invention, il va sans dire que l'invention étend aussi ses effets à des protéines hybrides obtenues par un couplage covalent de ses deux constituants principaux par l'intermédiaire d'une réaction chimique ressortissant à la chimie des protéines et permettant la liaison bout à bout desdits constituants, après protection adéquate des autres fonctions n'intervenant pas dans la réaction.

### RESULTATS

### Clonage et expression du gène CTB codant pour la sous-unite B de la toxine cholérique.

Nous avons cloné le gène CTB dans E. coli à partir du DNA génomique de la souche classique Ogawa 41 de Vibrio cholera obtenu de l'Institut Pasteur du Brabant. Le gène de la toxine cholérique a déjà été cloné (Gennaro et al., 1982; Pearson et Mekalanos, 1982) et séquencé (Lockman et Kaper, 1983; Lockman et al., 1984; Mekalanos et al., 1983a).

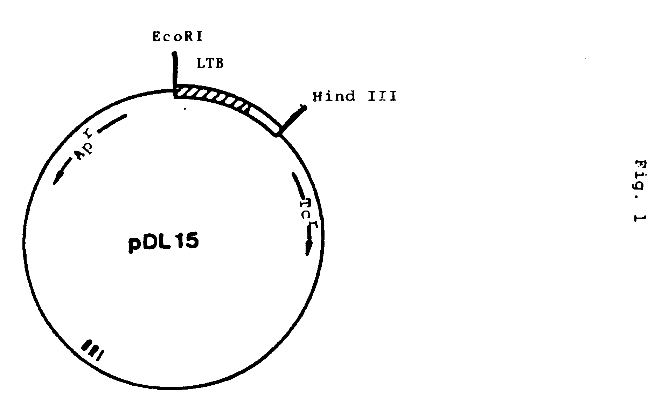

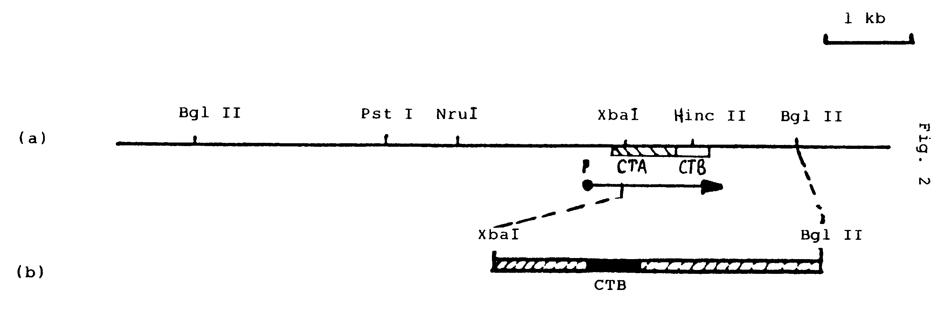

Pour suivre le gène de la toxine dans les différentes étapes du clonage, nous avons utilisé comme sonde la plasmide pDL15 (figure 1) qui contient sur un fragment EcoRI-HindIII le DNA codant pour la sous-unité B de l'entérotoxine "heat labile" d'E. coli (LTB). Le degré de similitude au niveau de la séquence nucléotidique avec le DNA CTB est de 77%. Nous avons reçu ce plasmide dans la souche E. coli MM294 du Dr. M. De Wilde de Smith Kline-RIT (Mekalanos et al., 1983a).

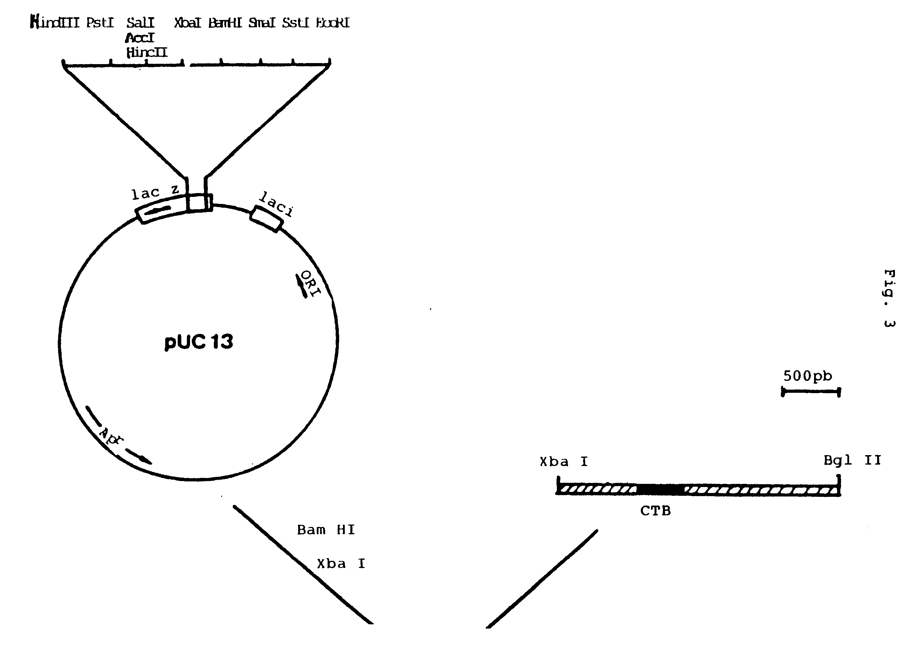

Les cartes de restriction des différents gènes de la toxine cholérique et des séquences adjacentes montrent que plusieurs sites sont conservés parmi les différentes souches de V. cholera (figure 2a). Sur la base de ces informations et aprés avoir vérifié la présence et la localisation de différents sites, nous avons choisi d'isoler le DNA CTB à partir d'un fragment XbaI-BglII du DNA génomique de V. cholera (figure 2a). Le site de restriction XbaI est situé au milieu du DNA codant pour la sous-unité A. Comme cette sous-unité est responsable de l'activité biologique, une toxine active ne peut être exprimée à partir de ce fragment et nous avons ainsi évité les risques de toxicité rencontrés lors du clonage du gène de la toxine entière.

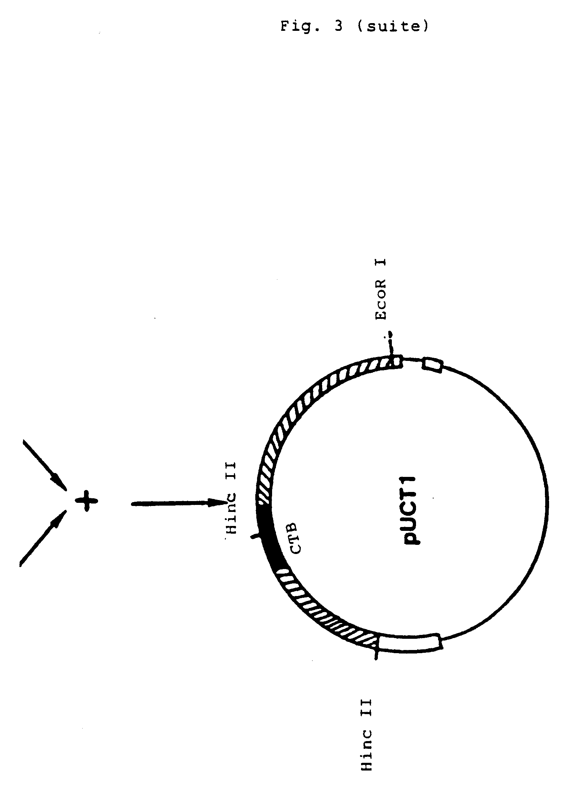

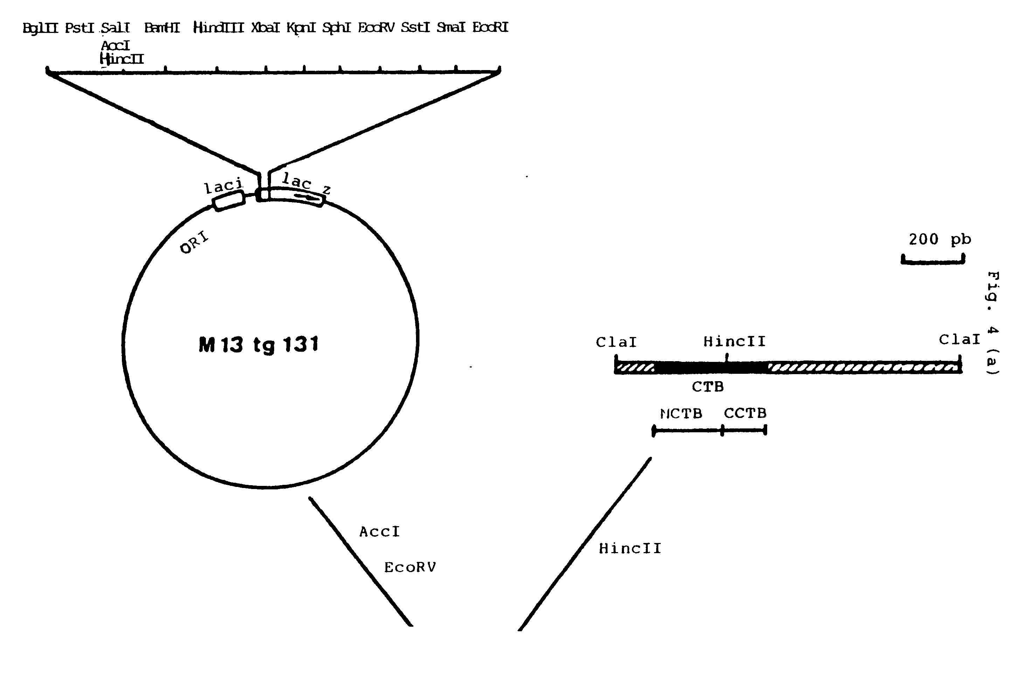

Le fragment XbaI-BglII a été cloné dans le plasmide pUC13entre les sites de restriction XbaI et BamHI (figure 3).

Le DNA génomique de la souche V. cholera ogawa 41 a été extrait suivant la méthode de Brenner et al. (1969) : après avoir lysé les bactéries par un traitement à la pronase (50 ug/ml) et au SDS (0.5%), le DNA a été extrait une fois au phénol en NaCl 0.4 M, SDS 1%, deux fois au phénol-chloroforme alcool isoamylique (25:24:1) et une fois au chloroforme alcool isoamylique seul (24:1); le DNA a finalement été précipité trois fois à l'éthanol sur une baguette de verre. Après un second cycle de traitement (pronase, SDS, phénol, éthanol), le DNA obtenu est suffisamment pur pour être digéré sans problème par les enzymes de restriction. A partir de 100 ml de culture de V. cholera, nous avons ainsi extrait 500 »g de DNA génomique.

La présence et la localisation des sites de restriction PstI, XbaI et BglII dans le gène de la toxine dans le DNA génomique le DNA de V. cholera a été vérifié par digestion avec un ou deux de ces enzymes. Les produits de ces digestions ont été séparés par électrophorèse sur gel d'agarose 0.7 % et transférés sur filtre de nitrocellulose. Les fragments contenant le DNA CTB ont ensuite été identifiés par hybridation avec le fragment EcoRI-HindIII contenant le gène LTB du plasmide pDL15I dans des conditions peu stringeantes, en 6XSSC, en 20 % formamide et à 37 C (Moseley et Falkow, 1980). La longueur des fragments a été estimée par comparaison avec les fragments du DNA du bactériophage lambda digéré par HindIII. Quelque soit la digestion, nous avons toujours observé deux bandes marquées. Ce résultat s'explique du fait que la souche classique de V. cholera, ogawa 41 contient deux copies bien séparées de l'opéron de la toxine (Mekalanos et al., 1983b). La longueur des fragments identifiés coincide avec la carte de restriction représentée dans la figure 2.

Pour le clonage du gène CTB, 30 »g de DNA génomique ont été digérés par les enzymes de restriction XbaI et BglII. Les fragments résultants ont été séparés par électrophorèse sur gel d'agarose 0.7 % et ceux dont la taille allait de 2.450 à 2.750 pb ont été récupérés par électroélution. Le mélange des fragments récupérés a ensuite été inséré entre les sites de restriction XbaI et BamHI du plasmide pUC13 (figure 3). Les nouveaux plasmides ont été introduite dans E. coli RRI∇M15. Les bactéries transformées par un plasmide contenant le gène CTB ont ensuite été sélectionnées par hybridation de colonies en conditions peu stringeantes avec la sonde LTB marquée au ³²P. Le marquage a été réalisé par transfert de coupure sur le fragment EcoRI-HindIII purifié du plasmide pDL15. Le DNA des plasmides des clones positifs ont ensuite été digérés par les enzymes de restriction EcoRI et HincII, les fragments résultant ont été séparés par électrophorèse sur gel d'agarose 1%, transférés sur filtre de nitrocellulose, hybridés avec le fragment EcoRI-HindIII du plasmide pDL15 marqué au ³²P et autoradiograhiés. Commele montre la figure 3. nous avons obtenu trois fragments de digestion (approximativement 890, 1660 et 2250 pb) et sur ces trois fragments, seuls ceux de 890 et 1660 pb qui contenaient une partie du DNA CTB se sont hybridés avec la sonde. Le plasmide que nous avons sélectionné a été dénommé pUCT1.

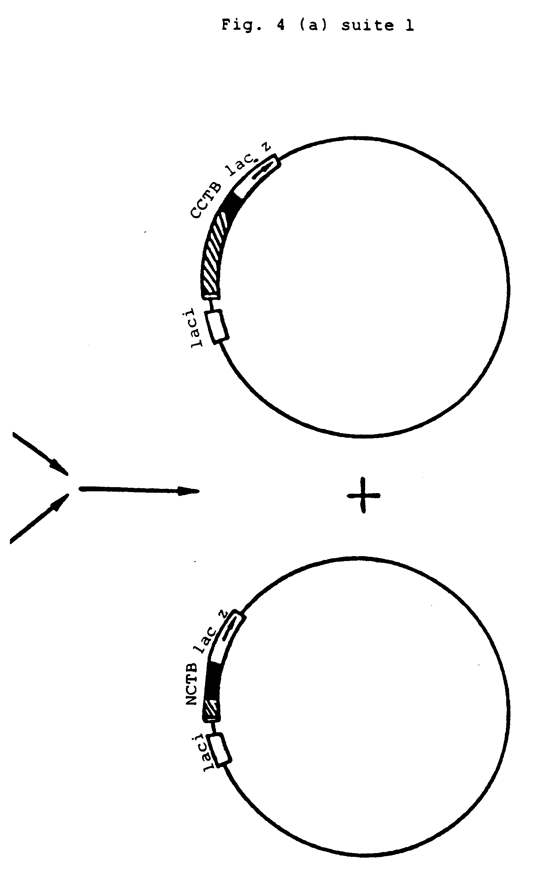

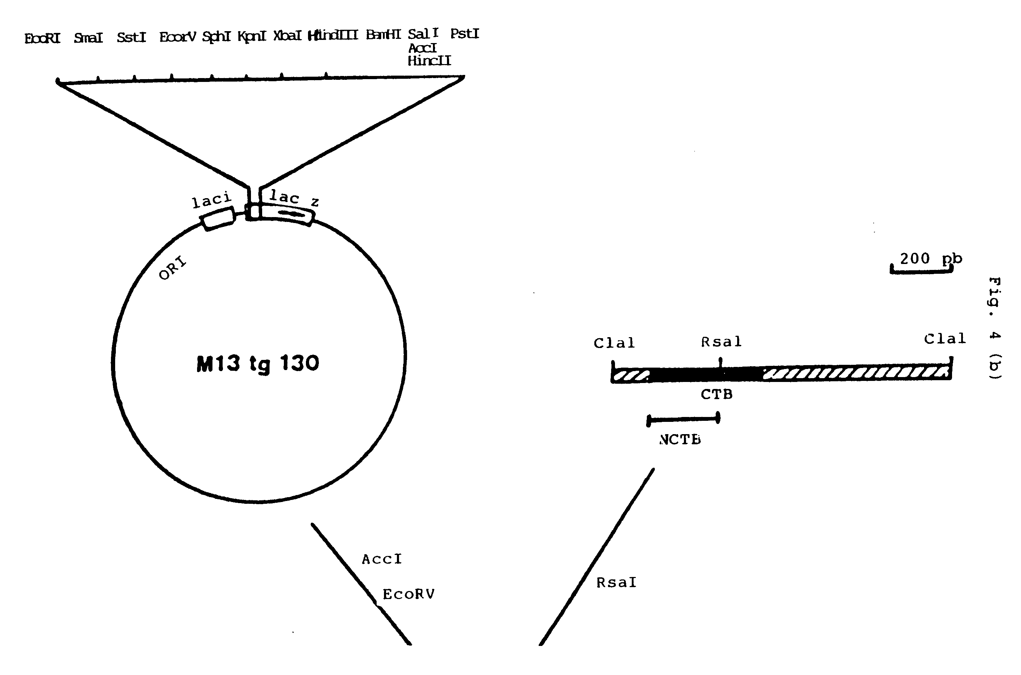

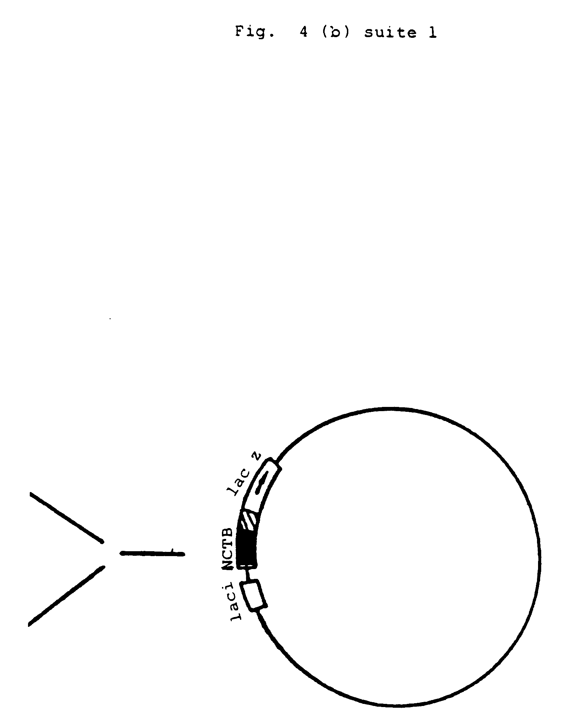

Nous avons ensuite déterminé la séquence du gène CTB par la méthode des "dideoxy" (Messing, 1983), après avoir recloné me fragment XbaI-BglII dans le bactériohphage M13 (figures 4a et 4b). D'après les séquences déjà publiées et d'après l'analyse par digestion enzymatique de pUCT1, nous avons isolé un fragment ClaI d'environ 1200 pb contenant l'entièreté du gène CTB et qui devrait commencer 138 pb en amont de l'ATG initial. Ensuite ce fragment a été digéré par l'enzyme de restriction HincII coupant au milieu du DNA CTB et les deux fragments résultant (approximativement 350, de préférence 370, et 800 pb) ont été insérés dans le DNA du bactérophage M13 to 131 entre les sites Accl (compatible avec le site ClaI) et EcoRV du polylinker (figure 4a). Un troisième fragment des 350 pb résultant de la digestion du fragment ClaI par l'enzyme de restriction RsaI a été inséré dans l'ADN du bactériophage M13 to 130 entre les sites AccI et EcoRV (figure 4b).

Les réactions de séquence ont tout d'abord été réalisées au [x³²P]dATP sur les trois matrices obtenues (DNA circulaire simple chaine) et ont ensuite été confirmées au [³⁵S] dATP[xS]. La séquence représentée dans la figure 5 est très similaire aux séquences des souches El Tor 2125 [Mekalanos et al., 1983a) et El Tor 62746 (Lockman et Kaper, 1983) à trois différences près (figure 6). Nous avons comparé la traduction en acides aminés avec quelques séquences primaires publiées (Nakashima et al., 1976; Lai 1977; Kurosky et al., 1977; Sela et al., 1984). Parmi ces séquences, les seules différences observées sont une glutamine à la place d'un acide aspartique et une serine à la place d'une valine (figure 6).

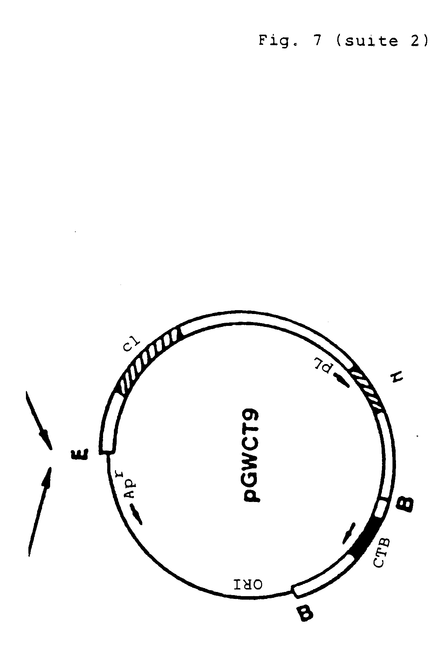

Le polypeptide synthétisé à partir du gène de CTB (124 acides aminés) contient une séquence signal qui n'est pas conservée dans la protéine mature de 21 acides aminés (figure 5). Chez V. cholera, cette séquence est nécessaire à la sécrétion de la protéine dans le milieu extérieur et. chez E. coli, elle permet la sécrétion dans l'espace périplasmique de la bactérie (Pearson et Mekalanos, 1982; Gennaro, 1982). Pour obtenir l'expression du gène CTB cloné, nous avons utilisé le vecteur d'expression pGW7 (figure 7). Ce vecteur porte le promoteur pL du phage lambda, qui est sous le contrôle négatif d'un répresseur thermo sensible, cI857. D'abord nous avons construit un vecteur plus petit (pGWM, 6251pb) en excisant le fragment BamHI-PvuII de pGW7 (figure 7). Pratiquement, le plasmide pGW7 a été digéré par les enzymes de restriction BamHI et PvuII et l'extrémité collante laissée par BamHI a été rendue franche par l'action du fragment de Klenow de la polymérase I d'E. coli. Le vecteur a ensuite été refermé sur lui-même avec la DNA ligase de T4 et introduit dans E. coli HB101. Cette construction nous a permis de créer un site unique de restriction BamHI au niveau de la jonction des deux anciens sites.

Ensuite, nous avons alors inséré dans le site BamHI du plasmide pGWM le fragment ClaI du plasmide pUCT1 contenant le gène CTB (figure 7). Le plasmide pGWM a été digéré par BamHI; les extrémités collantes obtenues ainsi que les extrémités du fragment ClaI ont été rendues franches par l'action du fragment de Klenow de la polymérase I d'E. coli; le mélange de ces deux fragments a ensuite été soumis a l'action de la DNA ligase de T4 et introduit dans E. coli; HB101. Le plasmide pGWCT9 possède le gène CTB dans la bonne orientation par rapport au promoteur pL (figure 7).

Nous avons mesuré l'expression du gène CTB dans le plasmide pGWCT9, en soumettant les bactéries contenant le plasmide à un shock thermique à 42°C qui inactive le répresseur cI857 thermosensible. Pratiquement, les bactéries transformées par les plasmides pGWM, pGWCT9 et pGWCT17 sont cultivées une nuit à 30°C; 110 ul de ces cultures sont alors dilués dans 5 ml de milieu; lorsque la densité optique à 600 nm atteint 0.4 C pendant 30 minutes et ensuite maintenues à 37 C pendant 90 minutes. Comme contrôle négatif, une fraction de ces cultures est maintenue à 30°C.

Dès l'induction, la multiplication des bactéries possédant le plasmide pGWCT9 (avec le gène de CTB dans la bonne orientation) s'arrête alors que le contrôle continue de se multiplier normalement. Ceci indique que la production de la protéine CTB bloque la multiplication bactérienne. L'analyse des protéines bactériennes par électrophorèse sur gel de polyacrylamide-urée 15% suivie d'une coloration au bleu de coomassie montre une fine bande entre 10.000 et 13.000 D légèrement plus intense dans les bactéries sensées exprimer CTB (P.M. 11.600).

### Prédiction de la structure de la sous-unité B de la toxine cholérique (CTB).

Dans notre prédiction de la structure tridimensionnelle de la protéine CTB nous nous sommes basés sur deux observations antérieures : (1) Fishman et al (1978) ayant démontré que la protéine est du type "all β" contenant essentiellement des régions avec une configuration beta (β-sheet) et (2) que la CTB forme des pentamères avec un seul axe de symmétrie d'ordre 5 (Divyer et Bloomfeld, 1982). Nous avons utilisé les algorithmes de prédiction de structure secondaire de Chou et Fasman (1978), de Cohen et al. (1986) et de Garnier et al. (1982) pour identifier les régions de structure secondaire dans la séquence d'acides aminés de la CTB. Nous avons retrouvé 8 régions distinctes de configuration beta séparées par des régions en configuration de boucle. Ce type d'organisation d'éléments de structures secondaires est typique de la famille des protéines dénommées "tonneau beta", où le noyau central de la protéine est formé par 8 chaînes en configuration β antiparallèles. En effet, il existe un ensemble de protéines dont la structure a été déterminée par analyse aux rayons X ayant exactement les mêmes caractéristiques: ce sont les protéines de la capside de certains virus de plante (Rossman et al., 1983) et des picornavirus (Hogle et al., 1985; Rossman et al., 1985).

Le coeur de ces protéines, fort similaire malgré l'absence d'homologie de séquences, est constitué par un tonneau de 8 brins β-antiparallèles en forme de "tranche de tarte", la pointe étant orientée vers l'axe d'ordre 5 (figure 8). Ces protéines sont toutes plus longues (150 à 300 acides aminés) que la sous-unité B (103 acides aminés). Dans ces protéines, une grande partie de la structure des boucles externes opposées à l'axe d'ordre 5 et une partie du tonneau central, sont responsables des zones de contact sur les axes d'ordre 3 et d'ordre 2 et assurent la stabilité de la capside virale, et sont donc spécifiques des ces protéines. D'autre part, une série d'autres protéines avec un tonneau β-antiparallèle, comme les différents domaines des immunoglobines, ont une taille comparable à CTB. Nous avons utilisé les règles d'empâquetage de Ptitsyn (1981) pour prédire la topologie du tonneain et le résultat de notre prédiction est représenté dans la figure 9 et que nous pouvons décrire comme suit : (1) Les 8 brins beta antiparallèles contiennent les résidus 3 à 5; 15 à 20; 24 à 27; 37 à 42; 46 à 52; 72 à 77; 86 à 88 et 95 à 100. (2) Des grandes boucles externes, exposées au solvant contiennent les résidus 6 à 14; 28 à 36; 53 à 71 et 89 à 94. (3) Les petites boucles internes de l'axe de symmétrie contiennent les résidus 21 à 23; 43 à 45 et 78 à 85. (4) Les deux extrémités NH₂ et COOH se trouvent au centre de l'axe de symmétrie et ne contiennent que quelques résidus : 2 à l'extrémité NH₂ et 3 à l'extrémité COOH.

Cette structure tridimensionnelle est en accord avec plusieurs observations antérieures : (1) les deux cystéines sont suffisamment proches pour former le pont disulfure (figure 9). (2) Les acides aminés impliquées dans la liaison avec GM1 (Dewolf et al., 1981) sont sur la même face de la protéine accessible au solvant. De même, les peptides antigéniques (Sela et al., 1984) sont sur cette face exposée au solvant (figure 9). (3) Les peptides non-antigéniques sont soit dans le tonneau, soit sur les boucles de l'axe de symmétrie. Finalement, l'organisation des brins "externes" et "internes" du tonneau est en accord avec la prédiction à l'aide du moment hydrophobique (Eisenberg et al., 1984).

Ce modèle de la structure tridimensionnelle de CTB nous a permis de définir des conditions préalables sous lesquelles nous pourrons utiliser la CTB comme protéine porteuse d'antigènes étrangers sans pour autant affecter ses propriétés d'attachement au récepteur cellulaire GM1. Comme l'attachement est probablement effectué par les boucles externes exposées au solvant, il en résulte que l'insertion d'un épitope d'antigène étranger dans une de ces boucles causerait vraisemblablement une interférence avec l'attachement au ganglioside GM1, soit directement en détruisant le site d'attachement, soit indirectement causant un empêchement stérique. Par contre, une insertion dans les boucles de l'axe de symmétrie devrait empêcher la formation du pentamère, pendant qu'une insertion dans les brins beta déstabiliserait la structure du tonneau. Il en résulte donc que probablement le seul endroit où nous pourrons attacher des chaînes d'acides aminés sans perturber la structure monomérique ou pentamérique et sans interférer avec son attachement au récepteur, sont en fait les deux extrémité NH₂ et COOH terminales(figure 9). De plus, comme les résidus 3 et 100 font partie de la structure centrale du tonneau, l'attachement de l'antigène étranger devrait se faire au delà de ces deux résidus si l'on veut préserver la structure tridimensionnelle. Vu les distances courtes entre les extrémités et les résidus 3 et 100, il nous a semblé prudent de laisser les deux premiers résidus NH₂ terminaux et les résidus COOH terminaux inchangés lors de l'attachement de l'épitope antigène étranger. En conclusion, notre prédiction de structure nous a permis de formuler une stratégie d'utiliser la protéine CTB comme protéine porteuse d'épitopes d'antigènes étrangers en couplant ces épitopes par voie génétique à la protéine CTB. En toute probabilité, il nous semble prudent d'opérer ce couplage aux deux résidus terminaux de la protéine CTB mature, bien que nous ne pouvons exclure qu'un couplage ne peut se faire respectivement aux résidus 2, 3, 100, 101 et 102.

### 2.3. Construction et expression de gènes CTB vecteurs.

En général, un couplage de protéines différentes par voie génétique peut être effectué au niveau des gènes codant pour les deux protéines en fusionnant les régions de DNA codantes respectives de façon telle qu'on obtient une seule région codante avec une phase de lecture non interrompue. La stratégie que nous avons utilisée consiste à construire des gènes CTB vecteurs dans lesquelles nous pourrons ultérieurement insérer des fragments de DNA codant pour des épitopes antigéniques. D'après le modèle de la structure tridimensionelle de CTB, on devrait pouvoir coupler des antigènes par voie génétique aussi bien à l'extrémité NH₂-terminale que COOH-terminale. En conséquence, nous avons décidé de modifier le gène CTB de façon telle d'avoir le choix de coupler des antigènes étrangers aussi bien à l'extrémité COOH-terminale que NH2-terminale. A cette fin, nous avons modifié les séquences codant pour les extrémités NH₂- et COOH-terminales du gène CTB de façon telle que les modifications de séquence introduites n'affectent pas la séquence primaire d'acides aminés de la protéine CTB mature, tout en créant des sites de restriction uniques :
- Dans la région NH-terminale nous avons introduit des modifications dans la séquence codante pour le peptide signal qui précède la protéine mature. De cette façon, nous pourrons obtenir des fusions dans une partie de CTB qui n'est normalement pas présente dans la protéine mature. En bref, nous avons raccourci de 13 acides aminés le peptide signal, ne retenant que 8 acides aminés. De cette façon le peptide de signal ne sera plus fonctionnel et devient un "leader peptide", long de 5 acides aminés. En même temps, nous avons introduit les sites de restriction BamHI et NlaIII dans le "leader peptide". C'est après l'ATG de ce leader peptide que nous pourrons insérer les fragments d'antigènes, et dans les protéines de fusion l'antigène sera suivi de la protéine mature CTB.
- Dans la région COOH-terminale, nous avons modifié la séquence de nucléotide codant pour les 10 derniers acides aminés : nous avons introduit le maximum de substitutions de nucléotides afin d'introduire un nombre de sites de restriction dans cette région, sans pour autant changer la séquence des acides aminés. En tout, nous avons pu créer 5 sites d'insertion : NlIII, PvuI, EcoRV, ClaI et BalI. Il est évident que lorsqu'un antigène sera couplé en insérant le gène dans un de ces sites de restriction la protéine de fusion résultante manquera quelques acides aminés, dépendamment du site d'insertion choisi. Comme les trois derniers résidus de l'extrémité COOH-terminale ne font pas partie de la structure du tonneau, il est concevable que la perte de ces acides aminés n'affectera pas les propriétés globales de CTB. Le gène CTB modifié sera par la suite dénommé gène CTB-vecteur.

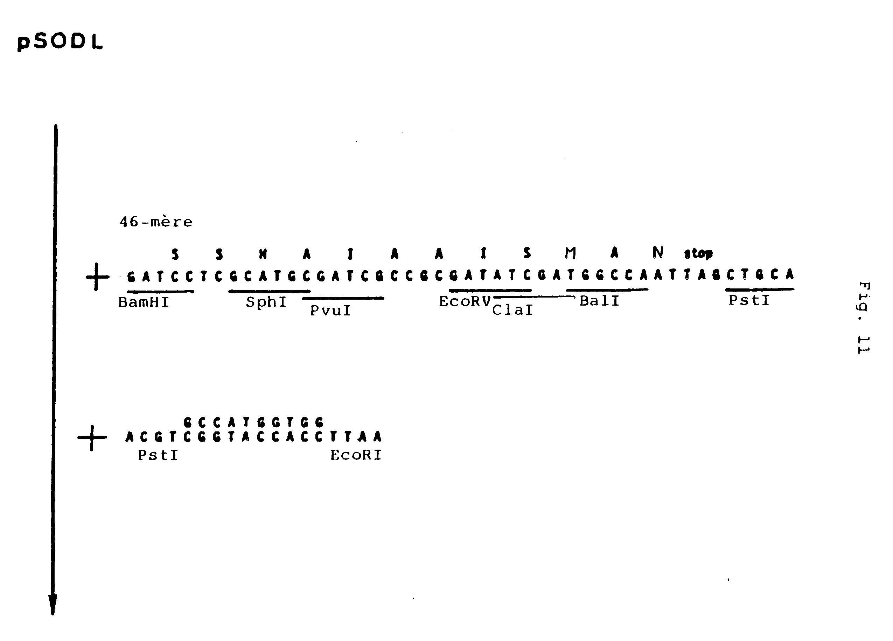

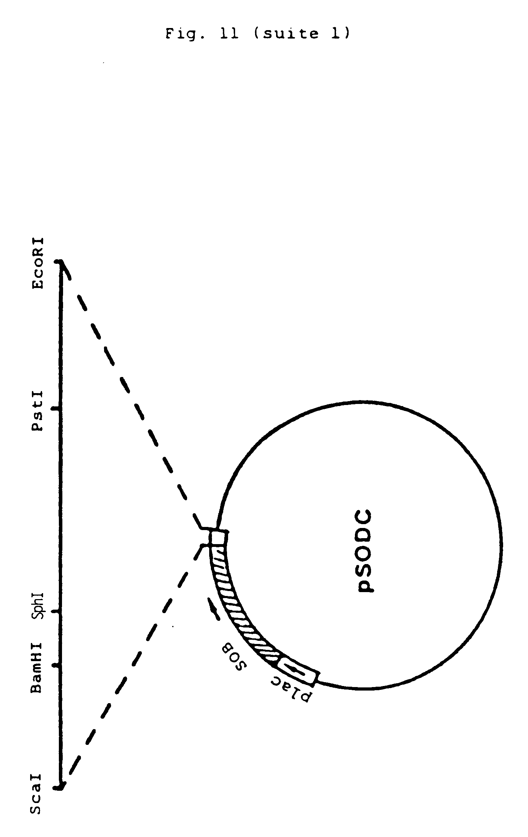

Pour obtenir l'expression du gène CTB-vecteur, nous avons utilisé la technique de couplage cistronique qui consiste à placer le gène derrière un autre gène déjà fortement exprimé de façon telle que lors de la traduction de RNA messager, ce gène sera traduit avec la même efficacité que le premier gène (Schoner et al., 1984). Ainsi, le gène CTB-vecteur sera couplé au gène de la superoxyde dismutase humaine hSOD dans un vecteur dérivé du plasmide pSODX16 (Hallewell et al., 1985). Le plasmide d'expression pSODX16 (Hallewell et al., 1985) contient le cDNA codant pour la hSOD sous le contrôle d'un promoteur fort, le promoteur "tac" inductible par l'IPTG (De Boer, et al., 1983). Dans le plasmide pSODL dérivé que nous avons construit, la fin de la séquence codante de la hSOD a été modifiée afin de permettre le couplage cistronique avec d'autre séquences codantes (figure 10). A cette fin, nous avons inséré un oligonucléotide contenant une séquence Shine-Dalgarno nécessaire à l'initiation de la traduction de la seconde protéine, un codon stop spécifiant la fin du cDNA de la SOD et un ATG pour l'initiation de la traduction du gène vecteur CTB suivi du site de restriction BamHI ou peut être inséré un fragment de DNA codant pour un épitope antigénique étranger (figure 10).

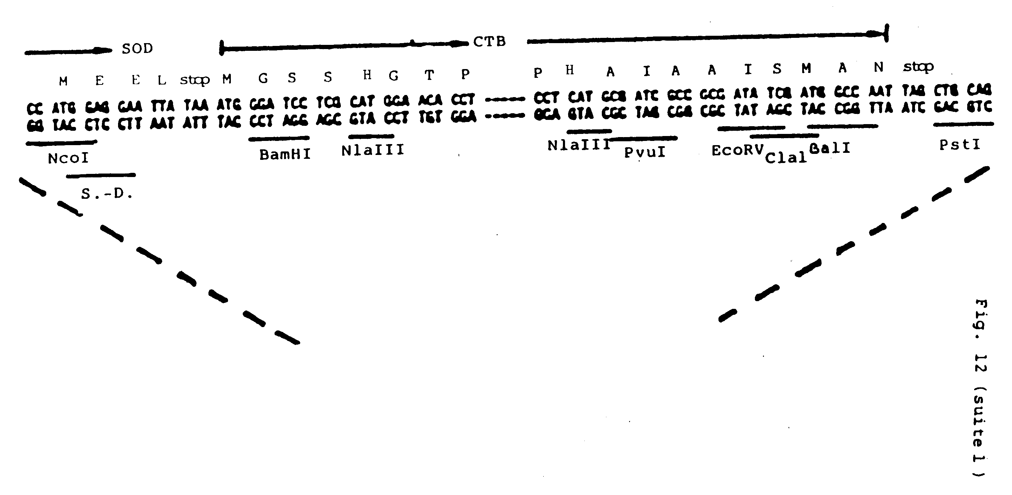

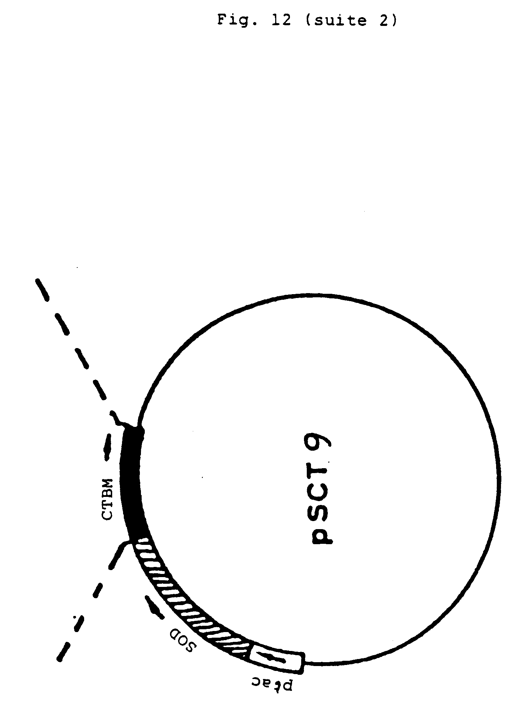

Avant d'insérer le fragment du gène CTB dans le plasmide d'expression, nous avons d'abord modifié le vecteur pSODL au moyen d'un oligonucléotide synthétique afin d'incorporer dans le plasmide les éléments génétiques nécessaires à obtenir la configuration du gène CTB-vecteur désiré. Le fragment du gène CTB qui sera inséré dans le vecteur est un fragment NlaIII, qui code pour la majorité de la sous-unité B mature excepté les 9 acides aminés C-terminaux et possédant en plus les 2 dernièrs acides aminés de la séquence signal (figure 12). Les 9 acides aminés qui manquent à l'extrémité COOH, ont été complété au moyen de cet oligonucléotide synthétique. En même temps, ceci nous a permis d'introduire les sites de restriction uniques, mentionés auparavant, qui permettront l'insertion ultérieure des fragments de DNA codant pour des épitopes antigéniques étrangers.

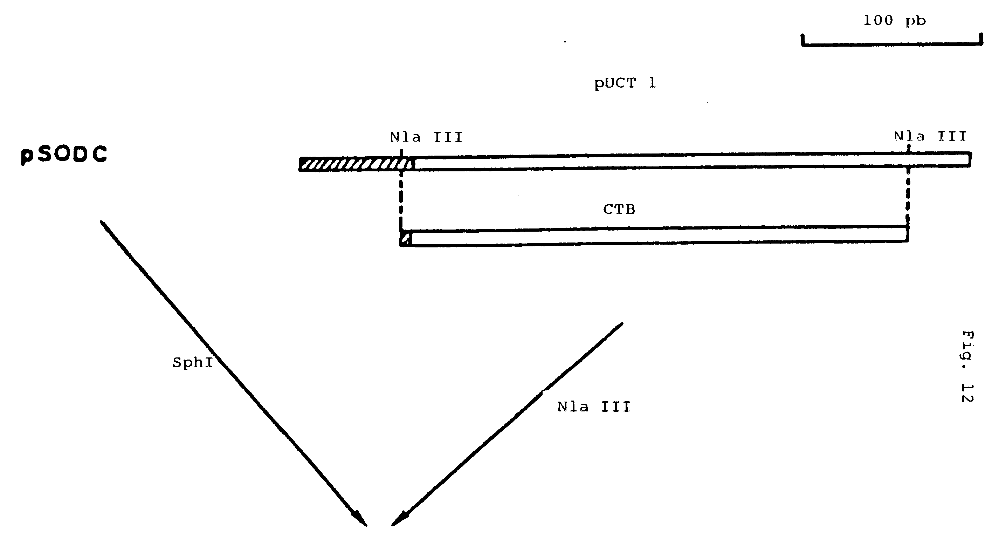

Le plasmide pSODC a été dérivé du plasmide pSODL par insertion de l'oligonucléotide synthétique à l'aide d'un adapteur PstI-EcoRI (figure 11). L'oligonucléotide dont la séquence est présentée dans la figure 11 contient successivement dans le sens 5'-3' : (1) une extrémité compatible avec le site de restriction BamHI; (2) un codon spécifiant une sérine. Les trois premiers acides aminés du gène vecteur CTB (méthionine, glycine et sérine) sont codés par le vecteur pSODL ainsi que le troisième nucléotide du quatrième codon. Comme nous utiliserons le site BamHI pour insérer les fragments, les résidus sérines pourront donner un couplage favorable dans la protéine chimère en plaçant deux sérines successives (Sung et al., 1986); (3) le site de restriction SphI où nous insérerons le fragment NlaIII; (4) le DNA codant pour les 9 acides aminés C-terminaux de CTB et le codon stop. La séquence primaire de CTB est inchangée et nous avons introduit des modifications dans la séquence nucléotidique afin de créer des sites d'insertion (figure 11); (5) une extrémité compatible avec le site de restriction PstI. La construction finale est représentée dans la figure 11. Pratiquement, la construction du plasmide pSODL a été accomplie comme suit : nous avons inséré l'oligonucléotide dans le plasmide pSODL au moyen du linker PstI-EcoRI. L'oligonucléotide (obtenu de Pharmacia) est d'abord purifié sur gel de polyacrylamide urée 16 %. Pour comparer la pureté de l'oligonucléotide avant et après cette étape, nous avons marqué une fraction au ³²P au moyen de la T4 polynucléotide kinase. Les produits obtenus ont été séparés sur gel de polyacrylamide-urée 16% et visualisés après autoradiographie. Ensuite, l'oligonucléotide a été purifié et phosphorylé à la polynucléotide kinase de T4, ajouté au linker PstI-EcoRI, et au vecteur digéré par BamHI et EcoRI dans un rapport de 1, 2 et 5 et soumis à la DNA ligase de T4. Nous avons traité le mélange de ligation avec le fragment de Klenow de la polymérase I afin de remplir les parties simple chaîne et nous avons ensuite transformé E. coli RRI M15 avec ces mélanges de ligation. Les bactéries transformées qui contiennent des plasmides ayant intégré l'oligonucléotide ont été détectées par hybridation avec l'oligonucléotide synthétique marqué au ³²P. Pour 25 ng de ligation, nous avons obtenu trois clones positifs.

L'étape finale dans la construction du gène CTB vecteur consiste à insérer le fragment CTB NlaIII dans le site SphI du vecteur pSODC (figure 12). Le fragment NlaIII a été obtenu en digérant le plasmide pUCT1 avec NlaIII et en purifiant le fragment de 300 paires de bases sur un gel d'agarose. Après avoir digéré le plasmide avec SphI, nous avons rajouté le fragment NlaIII purifié dans un rapport de 1 et soumis le mélange à la DNA ligase de T4. Ensuite nous avons transformé E. coli RRI M15 avec le mélange de ligation. Les plasmides présents dans les bactéries transformés sélectionés sur un milieu contenant 100 ug/ml d'ampicilline ont été analysé par digestion avec les enzymes de restriction BamHI, HincII et EcoRI afin d'identifier les bactéries transformées possédant le plasmide récombinant contenant le gène CTB-vecteur dans l'orientation désirée vis à vis du promoteur Tac. Un des plasmides ayant la structure désirée a été dénommé pSCT9 (figure 12).

Finalement, les constructions plasmides, la séquences de l'oligonucléotide synthétique ainsi que la séquence au niveau des sites de clonage dans le vecteur pSCT9 ont été vérifiés directement par séquençage de DNA à partir du DNA plasmidien par la méthode de Chen et Seeburg (1985). Pour cela, nous avons utilisé deux oligonucléotides synthétiques comme amorce : le premier reprend la fin de la séquence de la SOD, le second reprend la séquence du brin complémentaire situé en aval du DNA codant pour CTB. Les séquences du gène CTB-vecteur présentes dans la figure 11 ont ainsi été confirmées.

Nous avons analysé les protéines totales synthétisées par les bactéries possédant le plasmide pSCT9 après traitement à l'IPTG par électrophorèse sur gel de polyacrylamide-SDS 16% (PAGE-SDS) et coloration au bleu de coomassie. Nous n'avons pu mettre en évidence la présence de la protéine CTB vecteur et cela quelle que soit la durée de l'induction. Par contre, la présence de la protéine CTB-vecteur a cependant pu être détectée après transfert des protéines bactériennes séparées par PAGE-SDS sur filtre de nitrocellulose et révélation avec un antisérum spécifique dirigé contre la CTB commercialisé par Sigma (CTB Sigma). Nous avons aussi observé un ralentissement dans la vitesse de migration de la protéine CTB-vecteur, provoqué par la présence des 6 acides aminés supplémentaires. Finalement, le marquage à la Méthionine ³⁵S des protéines bactériennes exprimées aprés induction, suivi d'une chasse de durée variable avec de la méthionine friode, a permis de mettre en évidence la dégradation rapide de la protéine CTB-vecteur à l'intérieur de la bactérie.

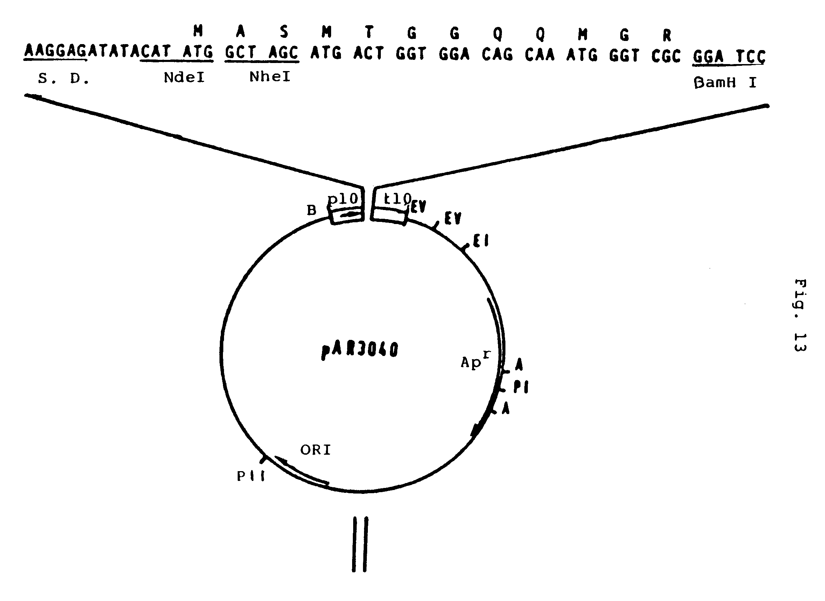

Suite à ces résultats, nous avons construit d'autres gènes CTB-vecteurs, qui codent pour des protéines CTB sans acides aminés additionels à l'extrémité NH₂ terminale. Pour ces nouveaux gènes CTB-vecteurs, nous avons utilisé un système d'expression différent basé sur l'utilisation de la RNA polymérase du bactériophage T7 (Studier et Moffatt, 1986). Le gène à exprimer sera placé dans le plasmide d'expression pAR3040 (figure 13) sous le contrôle du promoteur du gène 10 du phage T7. Le plasmide recombinant est alors introduit dans les bactéries BL21 (DE3) qui contiennent sur le chromosome une copie du gène de la RNA-polymérase du phage T7 sous contrôle du promoteur "lacUV5" inductible par l'IPTG. La RNA polymérase de T7 est très active et hautement sélective pour ses propres promoteurs, ce qui permet une rapide accumulation de la protéine désirée pouvant aller jusqu'à 50% des protéines totales bactériennes.

Comme dans tout système d'expression inductible, un faible niveau d'expression subsiste en absence d'inducteur. Ce système n'est donc applicable qu'aux protéines peu ou pas toxiques pour la bactérie. Pour l'étendre aux protéines plus toxiques, l'équipe de Studier y a introduit un autre plasmide, pLysS, qui permet l'expression du gène du lysozyme du phage T7 et qui est compatible avec le vecteur d'expression. Le lysozyme de T7 est un inhibiteur spécifique de la RNA polymérase de T7 et diminue donc le niveau d'expression du gène désiré en absence d'inducteur (Rosenber, Dunn et Studier, manuscrit en préparation).

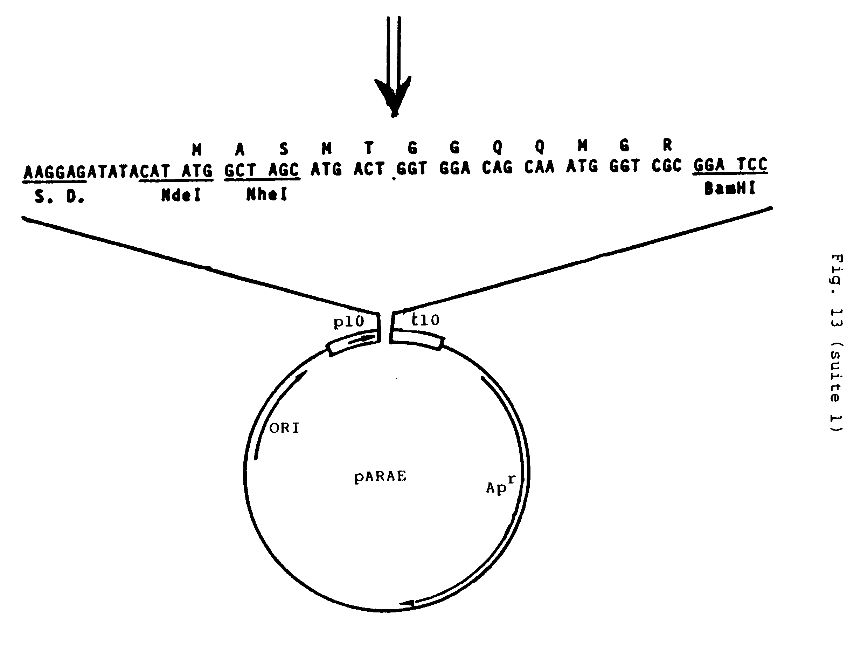

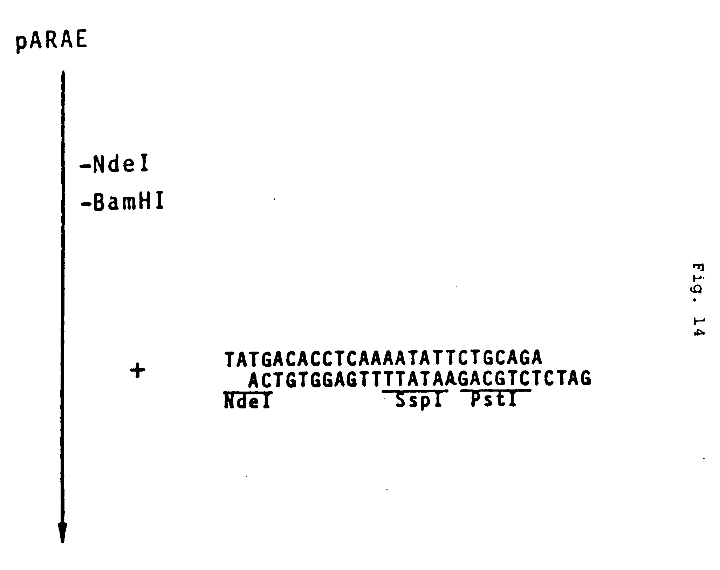

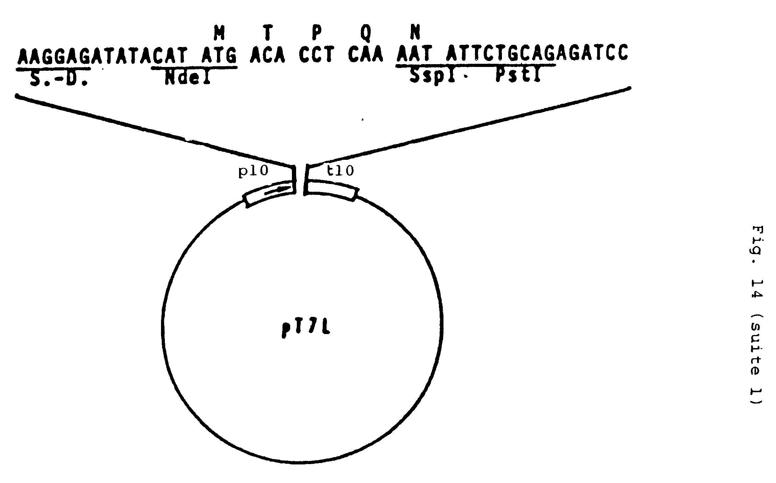

Le vecteur pAR3040 représenté dans la figure 13 est dérivé de pBR322 et contient le promoteur, la séquence Shine-Dalgarno et le terminateur du gène de la protéine 10 du phage T7. Le site de restriction NdeI localisé au niveau du codon d'initiation de la traduction de la protéine 10 nous permet d'insérer le gène CTB tout en conservant les séquences de T7 régulatrices de la transcription et les séquences nécessaires à sa traduction. Nous avons modifié le vecteur pAR3040 afin d'en réduire la taille du plasmide et d'éliminer des sites de restriction qui pourraient gêner les constructions ultérieures (figure 13) : 1. - les fragments PvuII-BglII et EcoRI-EcoRV ont été délétés et ces sites de restriction ont été supprimés; 2. - le site PstI situé dans le gène de résistance à l'ampicilline a été éliminé, en remplaçant le fragment AvaII de pAR3040 par le fragment équivalent du plasmide pUC13 dans lequel le site PstI a été muté. Le plasmide résultant a été dénommé PARAE. Nous avons construit le plasmide pT7CTBR qui contient le gène CTB-vecteur sous contrôle du promoteur T7 par insertion du fragment SspI-PstI du vecteur d'expression pSCT9 (figure 12) entre les sites NdeI et BamHI du plasmide pARAE. Avec un oligonucléotide synthétique, nous avons complété la séquence de CTB et nous avons réalisé les jonctions avec les sites d'insertions NdeI et BamHI. La construction est représentée figures 14 et 15.

Dans le plasmide pT7CTBR, les modifications qui avaient été introduites précédemment au niveau de la séquence nucléotidique de l'extrémité COOH-terminale de CTB lors de la construction de pSCT9 sont conservées. A l'extrémité NH₂-terminale, un seul codon a été ajouté pour permettre l'initiation de la traduction (figure 14). La séquence en acides aminés de la protéine vecteur CTB (CTBR) est donc identique à la séquence de la CTB mature naturelle excepté le résidu méthionine additionnel à l'extrémité NH₂-terminale (figure 15).

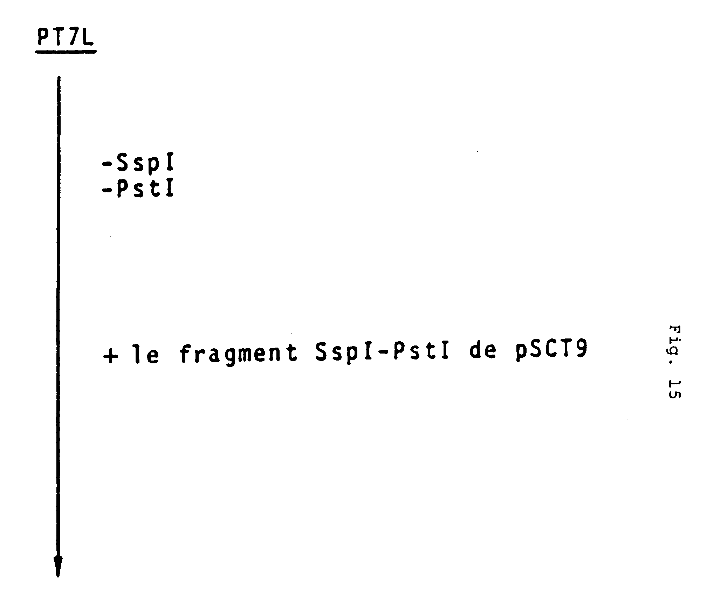

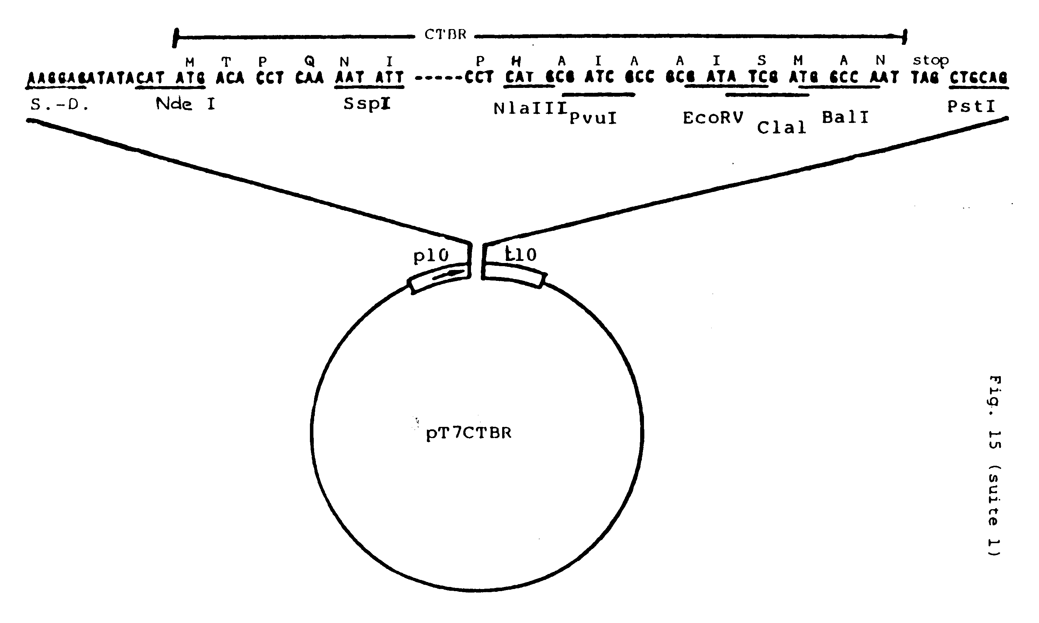

Ensuite, nous avons modifié le gène CTB-vecteur du plasmide pT7CTBR afin de créer un site d'insertion pour les fragments de DNA étranger dans la séquence de DNA codant pour l'extrémité NH₂ terminale du gène CTB-vecteur. Pour cela, nous avons introduit, dans le plasmide pT7CTBR, un site de restriction BamHI entre le 1er codon méthionine et le 2ème codon spécifiant le 1er acide aminé de CTB mature. Cette modification a été effectuée en remplaçant le fragment NdeI-SspI du plasmide pT7CTBR par un oligonucléotide synthétique et le plasmide résultant a été dénommé pT7CTBR2 (figure 16). Par rapport à la CTB mature, la nouvelle protéine CTB-vecteur (CTBR2) codée par pT7CTBR2 comporte 3 acides aminés supplémentaires à l'extrémité NH₂-terminale : successivement une méthionine, une glycine et une sérine.

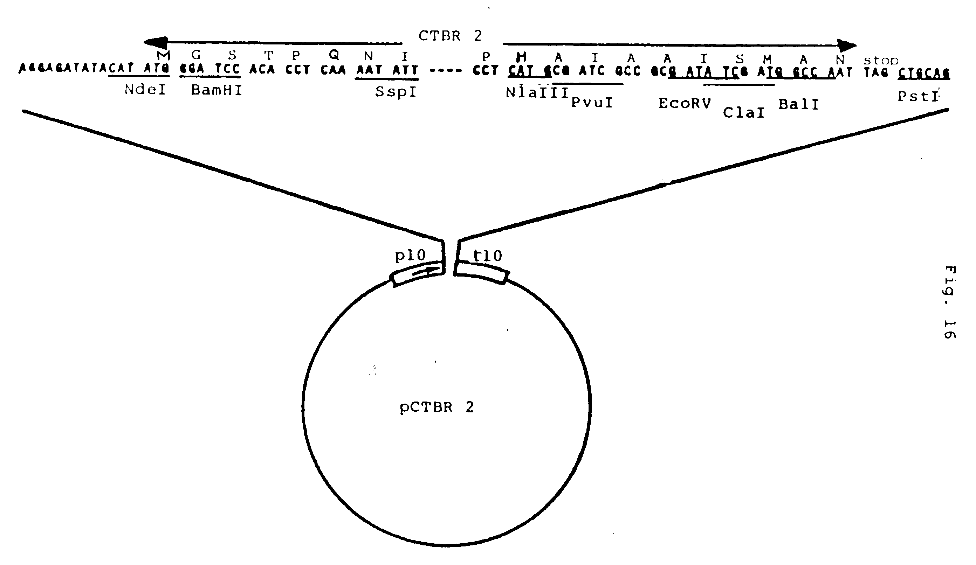

Nous avons obtenu des taux d'expression élevés des protéines CTBR (100 à 200 ug par ml de culture) dans la souche BL21 (DE3) transformée par pT7CTBR et pT7CTBR2 seul ou en présence de pLysS (figure 17). Cependant, nous avons constaté un fort ralentissement de la croissance cellulaire aprés induction dans le cas des bactéries possédant les deux plasmides contrairement aux cellules ne contenant que T7CTBR ou pT7CTBR2. La présence du plasmide pLysS permet d'obtenir un rapport favorable entre les protéines CTBR et les protéines totales (figure 17).

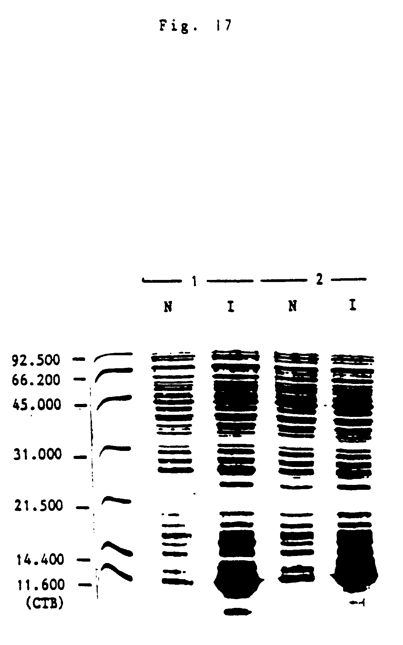

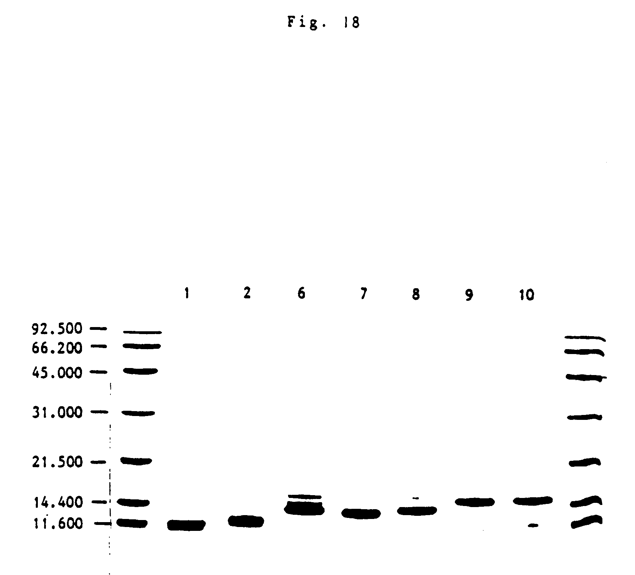

Nous avons observé qu'à l'intérieur de la bactérie, les protéines CTBR et CTBR2 s'aggrègent et forment des corps d'inclusion. Ce phénomène est fréquemment observé lorsque des protéines étrangères. sont exprimées chez E. coli (Uren, 1985). Ce phénomène a été mis à profit pour purifier les protéines CTBR et CTBR2 : les bactéries, concentrées par centrifugation, sont traitées successivement au lysozyme et à la DNAseI; les corps d'inclusion sont récupérés par centrifugation à 12.000 pendant 15 minutes et purifiés au cours de trois lavages successifs avec un tampon Tris HCl pH8.0 10mM, EDTA pH8.0 1mM PMSF 0,1 nM. L'analyse, par PAGE-SDS, des protéines présentes dans les corps d'inclusion montre que la totalité de CTBR et CTBR2 exprimée est récupérée dans ces aggrégats (figure 18).

### Activité biologique des protéines CTB vecteurs (CTBR et CTBR2)

La CTB forme naturellement un pentamère lorsqu'elle est sécrétée dans le milieu extérieur par V. cholera (Hirst et al. 1984; Gill, 1976). Par contre, les protéines CTB-vecteurs, CTBR et CTBR2 que nous exprimons à l'intérieur de la bactérie forment spontanément des corps d'inclusion qui contiennent des protéines agrégées insolubles. Comme la conformation pentamérique native de CTB est essentielle à son activité biologique de stimulation immunologique, nous avons recherché les conditions qui permettent de solubiliser les corps d'inclusion et d'obtenir ensuite la configuration pentamère. De plus, chaque sous-unité de la CTB naturelle possède un pont disulfure interchaine entre les cystéines en position 9 et 86 (figure 9). Comme nous ne connaissons pas l'état de réduction des protéines synthétisées dans E. coli, que nous avons exprimée, ce facteur a également été pris en considération dans l'établissement des conditions de rénaturation.

La formation du pentamère peut être mise en évidence par PAGE-SDS, le pentamère formant un complexe assez stable pour ne pas être dissocié en présence de SDS (Gill, 1976), pourvu que les échantillons ne soient pas chauffés à 100 C avant d'être déposés sur gel et pourvu que la concentration de SDS dans le tampon soit réduite à 0,1 %. Pour établir les conditions de rénaturation, nous nous sommes basés sur les conditions établies par Finkelstein et collaborateurs (1974) pour la reconstitution du pentamère CTB à partir de la toxine cholérique : les deux sous-unités peuvent être dissociées après dénaturation dans un tampon urée 6.5M, Glycine-HCl 0.1M pH3.2; le pentamère de CTB peut être reconstitué par dialyses successives à 4 C dans un tampon TrisHCl 50mM pH7.5 EDTA 10mM NaCl 200mM en présence de concentration décroissante d'urée (5M, 4M, 3M, 2M, 1M, 0.5M et 0M). Afin d'appliquer cette approche à nos corps d'inclusion, nous avons d'abord étudié leur solubilisation à différentes concentrations en urée et à différents pH : les résultats obtenus ont démontré qu'à pH 8, les corps d'inclusion sont solubilisés en urée 4M; par contre, à pH 3.2, les corps d'inclusion sont déjà solubilisés en urée 1M. Les corps d'inclusion de CTBM2 solubilisés en urée 1M ou 6.5M glyc 0.1M pH3.2 et dialysés successivement comme décrit par Finkelstein et al (1974). La dénaturation en urée 6.5M pH3.2 nous a permis de reconstituer le pentamère après dialyse. La solubilisation en urée 1M pH3.2 ne suffirait pas pour déplier complètement la chaîne polypeptidique de la protéine présente dans les corps d'inclusion nécessaire au repliement correct.

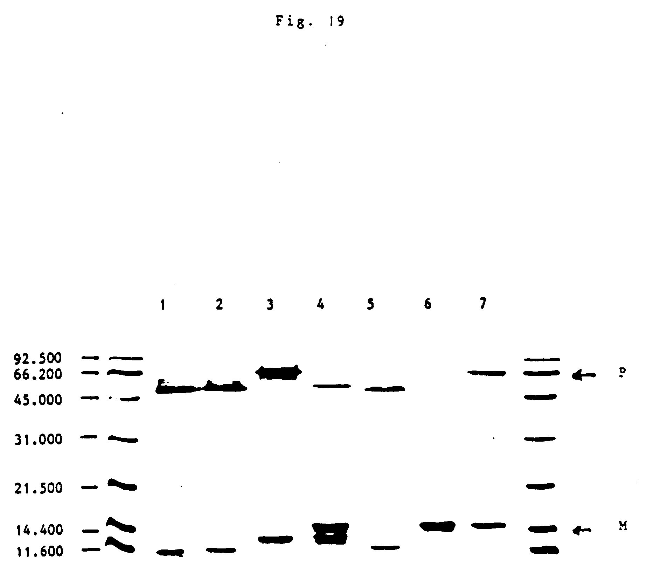

L'influence de l'état d'oxidation du pont disulfure intrachaîne sur la formation du pentamère a été analysée en appliquant le même cycle de dénaturation-renaturation en présence ou absence de β-mercaptoéthanol 1% dans le tampon de dénaturation. L'analyse par PAGE-SDS (figure 19) montre qu'en absence de β-mercaptoéthanol, en plus des pentamères de CTBR et CTBR2 migrant comme CTB Sigma (forme oxydée), nous observons des formes intermédiaires entre le monomère et le pentamère. Par contre, en présence de β-mercaptoéthanol dans le tampon de dénaturation, le pentamère de CTBR renaturé migre plus lentement (forme supposée réduite) et nous n'observons pas de formes intermédiaires. Il semble donc que l'oxydation des cystéines ait lieu, soit dans le cytoplasme de la bactérie, soit en cours de dénaturation. Cette oxydation provoquerait la formation de ponts de disulfures interchaînes (normaux) mais également de ponts disulfures interchaînes expliquant la présence de formes intermédiaires. Nous avons aussi analysé comment le rendement de renaturation en pentamère pouvait être influencé par le pH du tampon de dialyse. Les corps d'inclusion de CTBR solubilisés ont été dialysés contre des solutions de renaturation composées de NaCl 0.2 M, EDTA 1 mM, NaN₃ 3 mM, PMSF 0.1 mM et tamponnées soit en phosphate pH 6.0 100mM, soit en tris-HCl pH 7.5 100 mM, soit en Tris-HCl pH 8.5 100mM, soit en borate pH 9.5 100 mM. Nous avons étudié, d'une part, l'influence du pH lorsque les protéines sont dialysées directement contre le tampon de renaturation et d'autre part, lorsque les protéines sont dialysées successivement contre le tampon de renaturation contenant des concentrations en urée décroissantes. L'analyse par PAGE-SDS des protéines CTBR renaturées montre que, par dialyse par palier, le rendement est optimum à pH 7.5 et à pH 8.5. Par contre, par dialyse directe, le rendement optimal n'est obtenu qu'à pH 8.5 uniquement.

Nous avons finalement étudié parallèlement l'influence de la concentration des protéines et l'influence de la concentration en β-mercaptoéthanol dans le tampon de dialyse. Un précipité, composé de multimères formés par des ponts disulfures interchaînes, est observé à une concentration égale ou supérieure à 250 ug/ml lorsque le premier bain de dialyse, la précipitation n'est observée qu'à partir de 500 ug/ml ; l'importance du précipité est cependant plus faible lorsque le tampon ne contient pas de β-mercaptoéthanol. Les conditions de renaturation optimales sont donc une solubilisation des protéines dans un tampon contenant du β-mercaptoéthanol (1%) suivi de plusieurs dialyses par palier contre un tampon avec des concentrations décroissantes de β-mercaptoéthanol. En conclusion, nous pouvons donc obtenir un bon rendement des protéines CTB-vecteur (CTBR et CTBR2) à partir des corps d'inclusion purifiés en dénaturant d'abord les protéines en présence d'agent réducteur (β-mercaptoéthanol, 1%) et en présence d'une concentration d'urée 6,5 M dans un tampon glyc 0,1 M pH 3,2. La rénaturation se fait ensuite par dialyse par palier utilisant des concentrations décroissantes durée (5 M, 4 M, 3 M, 2 M, 1 M, 0,5 M et 0 M). β-mercapoéthanol est également ajouté aux deux premiers tampons de dialyse (respectivement 1% et 0.1%). De plus, ces résultats démontrent qu'il est possible de modifier l'extrémité NH₂-terminale de CTB sans affecter sa capacité de former un pentamère.

Ayant démontré que nous pouvons obtenir les protéines CTB-vecteurs sous la forme du pentamère ayant les mêmes propriétés physico-chimiques que le pentamère CTB naturel, nous avons ensuite vérifié la propriété des protéines CTB-vecteurs de s'attacher aux récepteurs gangliosides GM1. En effet, la fixation de CTB sur les récepteurs cellulaires, les gangliosides GM1 est responsable pour la stimulation de l'induction d'une réponse IgA immunitaire intestinale (McKenzie et Halsey, 1983). En plus, nous avons aussi vérifié si les propriétés antigéniques de CTB étaient conservées dans les protéines CTBR et CTBR2, en utilisant un antisérum polyclonal contre la protéine CTB.

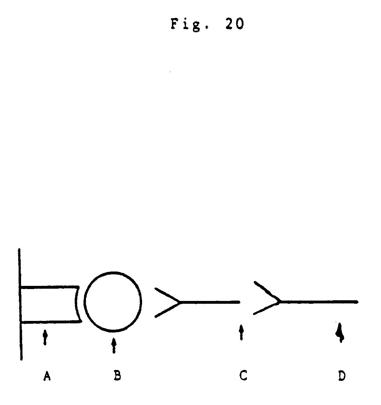

Pour étudier ces deux caractéristiques simultanément, nous avons utilisé un test GM1-ELISA illustré dans la figure 20 (GM1-Enzyme-Linked-ImmunoSorbent-Assay) dont les différentes étapes sont résumées ci-dessous (Lonroth et Holmgren, 1975; Sacks et al., 1980) : (1) adsorption de GM1 (obtenu de Sigma) sur les parois de microplaques; (2) fixation de CTB sur GM1; (3) reconnaissance de CTB par un antisérum de lapin dirigé contre CTB Sigma (antisérum anti-CTB); (4) reconnaissance des anticorps fixés par des anticorps de chévre anti-IgG de lapin couplés à la peroxydase (antisérum de révélation); (5) oxydation de l'ABTS (2.2'-Azinodi-(3-ethyl-BenzThiazolinSulfonate(6), sel diammonique) par la peroxidase en présence d'eau oxygénée; (6) mesure de la quantité d'ABTS oxydé par mesure de l'absorbance à 414 nm. Nous avons mis au point ce test avec la protéine CTB Sigma afin de déterminer les concentrations optimales des différentes solutions utilisées. D'après DeWolf et collaborateurs (1981), seul le pentamère est capable d'interagir avec les gangliosides GM1.

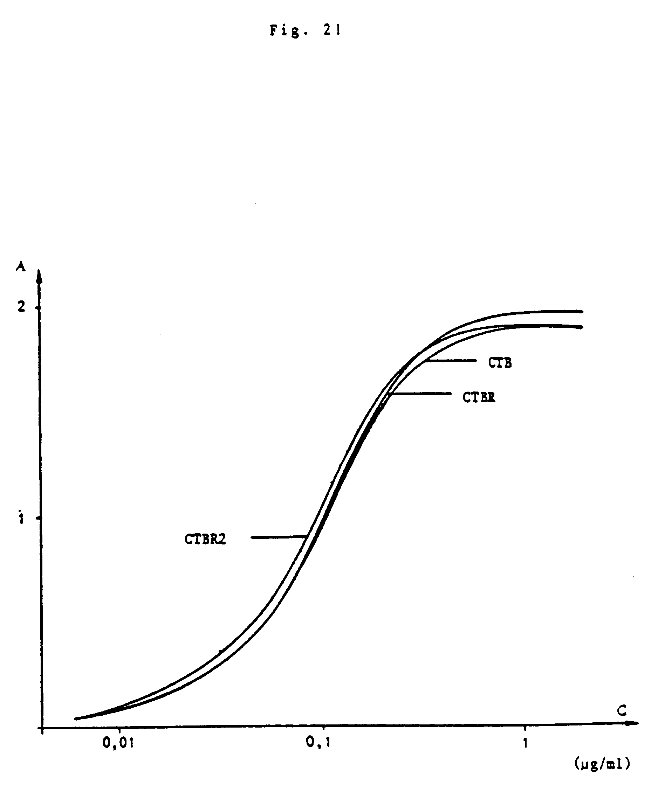

Le test GM1-ELISA a été appliqué aux protéines CTBR et CTBR2 renaturées en ne tenant compte que de la concentration en forme pentamèrique. Pour estimer cette concentration, les formes pentamèriques des différentes protéines renaturées ont été comparées à une quantité connue de CTB Sigma après PAGS-SDS et coloration au bleu de coomassie. CTB Sigma sert également de référence dans ce test. Les courbes obtenues dans le test GM1-ELISA pour les formes renaturées des protéines CTBR et CTBR2 sont identiques à celle de CTB Sigma (figure 21). Ces résultats démontrent que les deux protéines CTB vecteurs, CTBR et CTBR2, ont des propriétés d'attachement aux récepteurs GM1 identiques à celles de la protéine CTB naturelle. Il en résulte que les 3 acides aminés supplémentaires à l'extrémité NH₂₋ terminale de CTBR2 n'interfèrent pas avec la formation du pentamère et n'influencent pas non plus la réactivité avec les gangliosides GM1 et avec l'antisérum anti-CTB.

### Construction et expression des protéines de fusion entre CTBR2 et des épitopes antigéniques

Nous avons utilisé la stratégie suivante dans la construction des protéines de fusion : chaque épitope antigénique a été couplé à la protéine CTB-vecteur soit à l'extrémité amino terminale, soit à l'extrémité COOH terminale. Les couplages NH₂ terminaux ont été réalisés en insérant des fragments de DNA codant pour l'épitope dans le site BamHI en aval de ATG initiateur du vecteur pT7CTBR2,tandis que les couplages COOH terminaux ont été réalisés en insérant les fragments de DNA dans le site Bal1 de pT7CTBR ou entre le site BglII et BamHI du vecteur pT7CTBR5. Nous avons fait des couplages témoin en insérant le fragment au milieu de la protéine CTB-vecteur, plus précisément entre les acides aminés sérine 55 et glutamine 56. Ainsi les protéines de fusion témoins incorporent l'épitope antigénique dans une des boucles externes, présumées d'être impliquées dans l'interaction avec le récepteur GM1. Ces constructions témoin ont été réalisées en insérant les fragments de DNA dans le site HincII du plasmide pT7CTBR ou dans le site BglII et BamHI du vecteur pT7CTBR4. La construction et l'amplification des différents plasmides d'expression ont été réalisées dans les bactéries E. coli HB101. Les plasmides ont ensuite été transférés dans les bactéries BL21(DE3) pour obtenir l'expression des différentes protéines hybrides.

### Fusion entre CTBM3 et le fragment F1 de la protéine E2 du virus de la gastroentérite de porc

Le premier antigène que nous avons utilisé est l'antigène de surface du virus de la gastroentérite transmissible du porc (GET), virus appartenant aux coronaviridae, une famille de virus enveloppés à RNA monocaténaire linéaire de la polarité positive. Ce virus provoque chez le porcelet une maladie sévère et contre laquelle les essais de vaccination se sont montrés inefficaces avec des virus inactivés ou tués. Comme les coronavirus purins et aviaires, les virions GET possèdent trois protéines : une protéine N qui avec le RNA forme la nucléocapside et deux protéines d'enveloppe glycosylées, E1 (PM 29000) et E2 (220000). Alors que E1 est essentiellement transmembranaire, E2 forme, à la surface du virus, des projections appelées peplomères. Les anticorps neutralisants apparaissent spécifiquement dirigés contre ces peplomères indiquant que la protéine E2 est un bon candidat comme antigène protecteur (Siddell et al., 1983). Cette protéine est composée de 1447 acides aminés. La topographie des épitopes a été établie à l'aide d'une batterie d'anticorps monoclonaux (Delmas et al., 1986). Un fragment F1 de 150 acides aminés a été identifié étant capable de réagir avec les antiséra reconnaissant le site antigénique C, dont l'antisérum monoclonal 3b5.

Le cDNA du virus a été cloné et partiellement séquencé (Rasschaert et al., 1987). A partir de clone contenant un fragment de cDNA codant pour la protéine E2, nous avons isolé le fragment F1 de 451 nucléotides par digestion avec les enzymes de restriction XhoI-HpaI (cDNA-F1). Le plasmide pUCF1 a été obtenu par l'insertion de ce fragment dans le site SmaI de pUC13. Ensuite nous avons isolé le fragment F1 du plasmide pUCF1 et puis le fragment a été inséré aux trois sites de restriction précités. Les jonctions ont été réalisées avec un oligonucléotide synthétique de manière à conserver la phase de lecture dans les trois gènes hybrides.

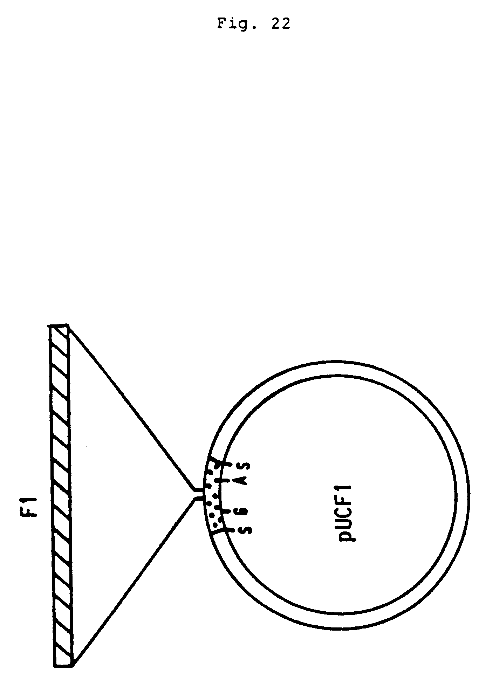

La stratégie que nous avons adoptée est représentée figure 22. Les nouveaux plasmides sont dénommés pCLHG1, pCLHG2 et pCLHG3. Nous avons transformé les bactéries BL21(DE3)-pLysS avec les différents plasmides que nous avons construits. Outre l'avantage d'utiliser un oligonucléotide synthétique unique pour les trois constructions, cette stratégie a permis d'insérer les sites de restricion BglII et BamHI aux anciens sites HincII et BalI de pT7CTBR. Ces sites étant dans la même phase de lecture que le site BamHI de pCLH2, une stratégie de clonage unique a pu et pourra être utilisée pour réaliser parallèlement les trois fusions dans des constructions ultérieures.

Les protéines CLHG1, CLHG2 et CLHG3 sont respectivement codées par les plasmides pCLHG1, pCLHG2 et pCLHG3 et résultent de la fusion génétique entre le protéine CTB-vecteur et le fragment F1 de la protéine E2 du virus de la GET. Après induction de l'expression de ces protéines dans les bactéries BL21(DE3) nous avons observé la formation de corps d'inclusion. Ce phénomène a été mis à profit pour purifier les protéines de fusion. Les bactéries concentrées par centrifugation, sont traitées successivement au lysozyme et à la DNAse I. Les corps d'inclusion sont récupérés par centrifugation à 12000g pendant 15 minutes et purifiés au cours de trois lavages successifs avec un tampon tris HCl pH 8.0 10 mM, EDTA pH 8.0 1 mM, PMSF 0.1 mM. L'analyse par PAGE-SDS des protéines totales et des protéines présent dans le corps d'inclusion montre que la totalité des protéines hybrides exprimées se retrouvent dans ces aggrégats. Comme le rapport protéines hybrides/protéines totales est plus faible qu'avec CTBR, nous avons traité les corps d'inclusion pendant une nuit avec des solutions durée en concentration variable tamponnées à pH 7.5 avec du tris HCl 100 mM afin de réduire la présence de protéines contaminantes. Nous n'avons pas réussi à solubiliser les trois protéines à partir des corps d'inclusion, du moins dans le présent essai et avec la solution tamponnée choisie.

### Fusion de CTBM3 et l'épitope 3b5 de la protéine E2 du virus de la GET.

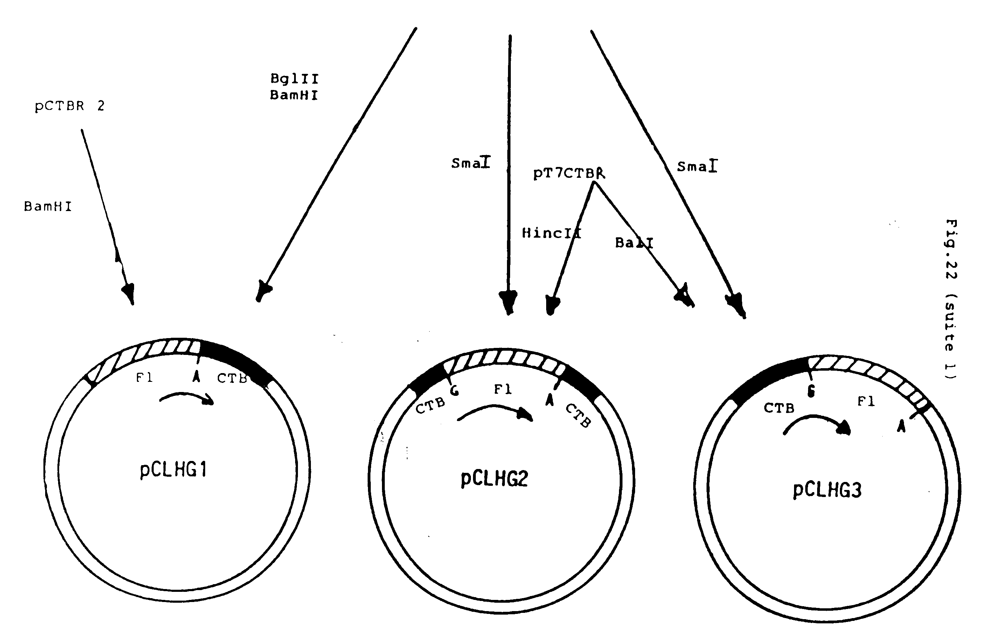

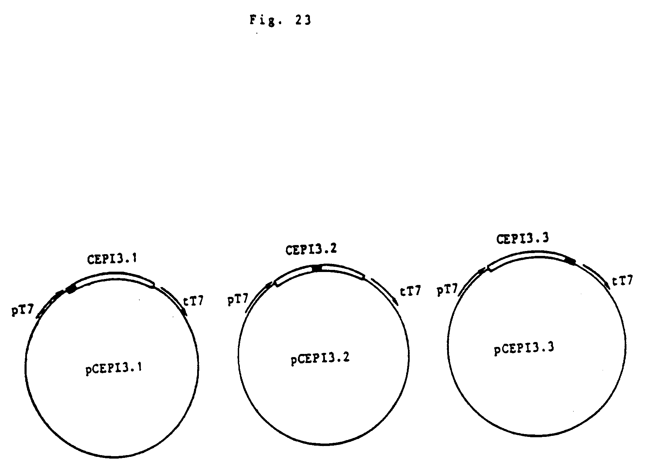

L'équipe du Dr. H. Laude a localisé plus précisément sur le fragment F1 la séquence reconnue par l'anticorps monoclonal 3b5. Nous avons inséré dans les gènes CTB vecteur le cDNA codant pour cet épitope linéaire de 9 acides aminés, plus les deux acides aminés localisés en amont et l'acide aminé localisé en aval. Ces codons supplémentaires ont été ajoutés afin de mieux séparer l'épitope antigénique et la partie CTB dans les protéines chimères. Le DNA codant pour cette séquence auquel ont été ajoutés des sites BglII et BamHI aux extrémités a été synthétisé chimiquement. Cet oligonucléotide a été introduit dans le site BamHI de pT7CTBR2 et entre les sites BglII et BamHI de pT7CTBR4 et de pT7CTBR5. La structure des plasmides est présentée dans la figure 23. Les trois protéines hybrides CEPI3.1, CEPI3.2 et CEPI3.3 codées par les plasmides, pCEPI3.1, pCEPI3.2 et pCEPI3.3, possèdent ainsi l'épitope inséré respectivement à l'extrémité NH₂-terminale, au centre et à l'extrémité COOH-terminale de CTB.

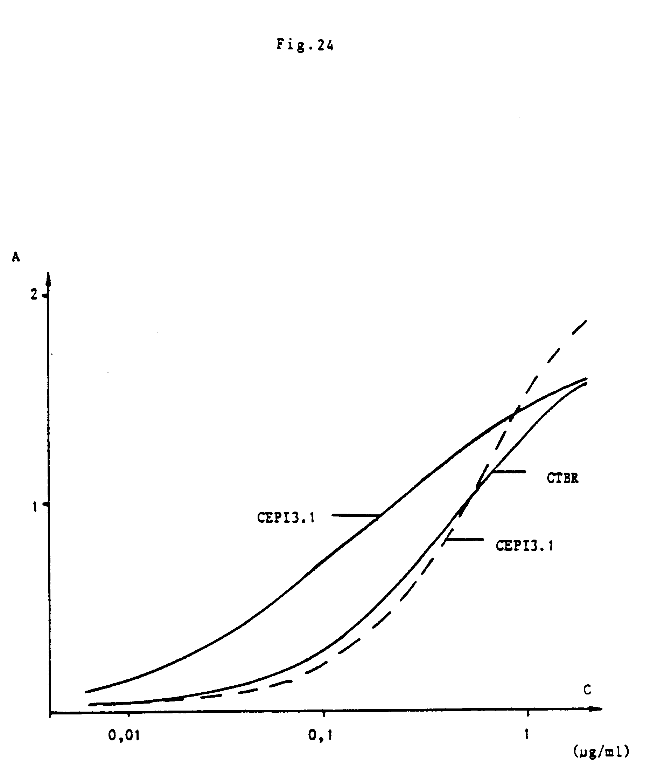

Les trois protéines ont pu être exprimées à haut taux par les bactéries BL21(DE3) contenant le plasmide pLysS et les plasmides respectifs, les taux étant similaires à celui observé pour l'expression de CTBR ou CTBR2. Les protéines de fusion ont été purifiées à partir des corps d'inclusion comme décrit avant. L'analyse des protéines présentes dans les corps d'inclusion par PAGE-SDS est illustré dans la figure 18. Nous avons utilisé les conditions de renaturation établies précédemment et nous avons pu reformer un pentamère soluble avec une des trois protéines fusions, CEPI3.1 (insertion de l'épitope a l'extrémité NH₂-terminale de CTB)(figure 19). Lorsque l'épitope est inséré au centre de CTB (CEPI3.2), la protéine est solubilisée mais n'est pas polymérisée en pentamère. La troisième protéine, CEPI3.3, où l'épitope est localisé a l'extrémité COOH-terminale de CTB, une faible proportion est solubilisée sous forme de monomère mais la majorité se retrouve sous forme d'un précipité. En conclusion, nous avons pu reconstituer le pentamère natif lorsque l'épitope viral 3b5 est inséré à l'extrémité NH₂-terminale. Nous avons utilisé le test GM1-ELISA pour vérifier si la protéine de fusion CEPI3.1 possédait toujours la capacité à se fixer sur les récepteurs cellulaires GM1, et était reconnue de la même manière que la CTB naturelle par un antisérum polyclonal dirigé contre la CTB. Ce test a démontré que la protéine CEPI3.1 conservait la propriété de CTB de se fixer sur les gangliosides GM1 et était toujours reconnue par l'antisérum anti-CTB (figure 24). Nous avons aussi effectué le même test GM1-ELISA où l'antisérum anti-CTB était remplacé par l'anticorps monoclonal 3b5 reconnaissant l'épitope (figure 24). Nous avons ainsi montré que l'anticorps monoclonal reconnaissait l'épitope sur la protéine hybride, elle-même préalablement fixée au récepteur cellulaire, le ganglioside GM1. Comme contrôle négatif,nous avons également montré que cet anticorps monoclonal ne reconnaissait pas la protéine CTBR.

### Fusion de CTBM3 et l'épitone C3 du virus de la poliomyélite

L'épitope C3 du virus de la poliomyélite est un peptide hydrophile localisé entre les acides aminés 93 et 103 de la protéine de capside VP1 (Wychowsky et al., 1983) et est reconnu par un antisérum monoclonal neutralisant (C3). Ce même épitope a été utilisé pour rechercher des sites d'insertion pour des antigènes étrangers dans le récepteur du phage lambda, une protéine de la membrane externe d'E. coli (Charbit et al., 1986).

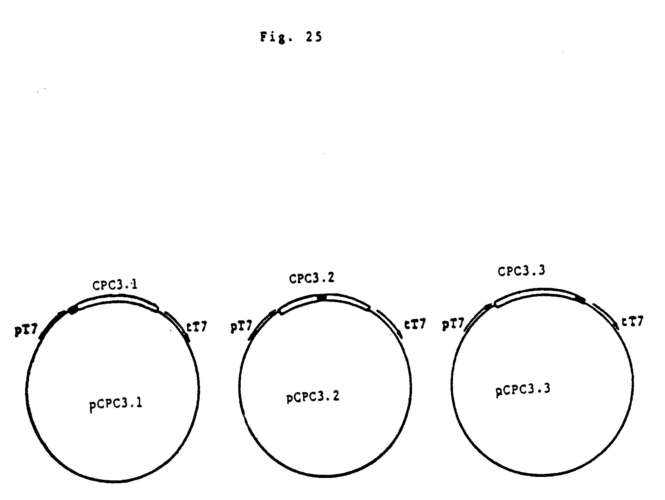

Dans la conception de l'oligonucléotide codant pour l'épitope C3 de polio, nous avons ajouté des séquences codant pour des acides aminés susceptibles de former des boucles de chaque côté de la séquence codant pour l'épitope. Nous avons introduit cette modification afin d'obtenir des protéines de structure pentamérique lorsque l'étiope est inséré à l'extrémité COOH-terminale ou au centre de CTB. La séquence de l'oligonucléotide que nous avons synthétisé pour réaliser ces nouvelles constructions, ainsi que la séquence en acides aminés correspondante, sont représentées figure 25. Cet oligonucléotide a été insérée au site BamHI de pT7CTBR2 et entre les sites BglII et BamHI de pT7CTBR4 et de pT7CTBR5. Les trois nouveaux vecteurs obtenus ont été dénommés pCPC3.1, pCPC3.2 et pCPC3.3.

Les protéines CPC3.1, CPC3.2 et CPC3.3 sont codées respectivement par les vecteurs pCPC3.1, pCPC3.2 et pCPC3.3 et résultent de la fusion génétique entre CTB et l'épitope C3 du virus de la poliomyélite Seules les protéines CPC3.2 et CPC3.3 ont pu être exprimées à haut taux (insertion au centre et à l'extrémité COOH-terminale de CTB). Lorsque l'expression de CPC3.1 est induite, les bactéries arrêtent de croître similairement aux bactéries exprimant CTBR ou CPC3.2 et CPC3.3 mais aucune protéine exprimée ne peut être mise en évidence après PAGE-SDS et coloration au bleu coomassie. Nous avons purifié les protéines CPC3.2 et CPC3.3 à partir des corps d'inclusion (figure 18) et nous avons rénaturé les protéines solubilisées Les résultats présentés dans la figure 19 montrent que dans les conditions particulières choisies pour l'expérience seule la protéine CPC3.3 (couplage COOH-terminal) forme un pentamère.

La capacité de ces différentes protéines fusion à se fixer aux récepteurs cellulaires GM1 a été étudiée au moyen du test GM1-ELISA décrit précédemment. Dans le test GM1-ELISA, nous avons utilisé comme premier anticorps soit un antisérum polyclonal anti-CTB (figure 26), soit l'anticorps monoclonal 3b5 qui reconnaît l'épitope du virus de la GET que nous avons inséré dans CTB (figure 27), soit l'anticorps monoclonal C3 qui reconnaît l'épitope C3 du virus de la poliomyélite (figure 28). Dans les deux derniers cas, nous avons utilisé comme anticorps de révélation des anticorps de lapin anti-IgG de souris couplés à la péroxydase.

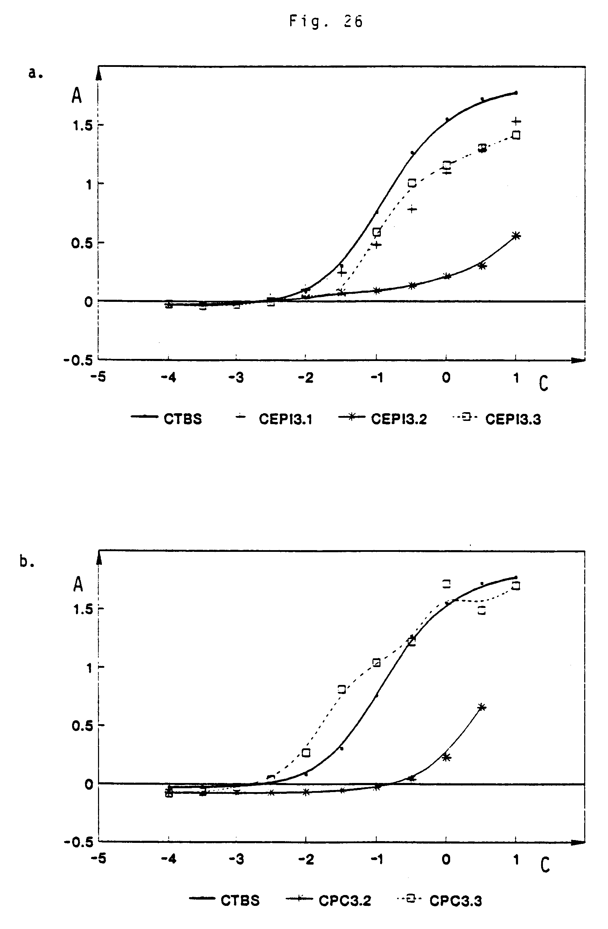

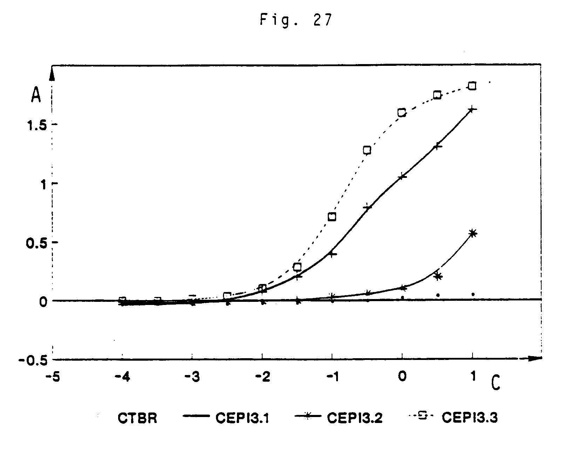

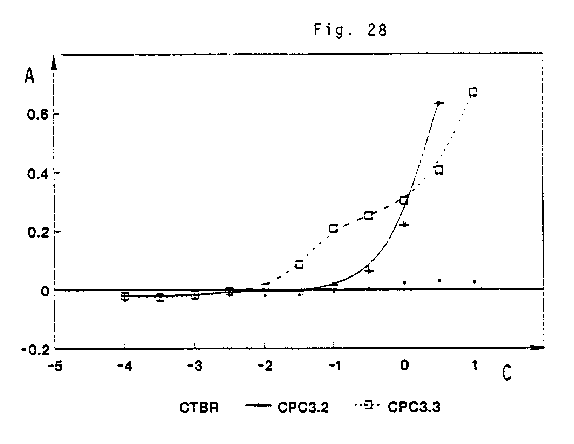

D'après Dewolf et collaborateurs (1981), seul le pentamère est capable d'intégrer avec les gangliosides GM1. Dans les préparations de protéines hybrides renaturées, une proportion variable de forme monomérique subsiste. Les différents tests ont été réalisés en ne tenant compte que de la concentration en forme pentamérique. Cette concentration a été estimée en comparant les formes pentamériques des différentes protéines à une quantité connue de CTB Sigma après PAGE-SDS et coloration au bleu de coomassie. CTB Sigma sert également de référence dans le test. Les résultats du test GM1-ELISA anti-CTB (figure 26) indiquent qu'en général les protéines hybrides résultant de la fusion NH₂- ou COOH-terminales de l'épitope viral à CTB (protéines CEPI3.1, CEPI3.3, CPC3.3) conservent la capacité de CTB à se lier aux récepteurs GM1. De plus, le test GM1-ELISA anti-épitope (figures 27 et 28) montre que lorsque l'épitope viral est fusionné aux extrémités NH₂- ou COOH-terminales de CTB, celui-ci est toujours reconnu par l'Ac monoclonal spécifique, après fixation de la protéine hybride aux récepteurs GM1.

En conclusion, ce test nous permet de dire que lorsqu'un épitope viral est fusionné à l'une ou l'autre extrémité de CTB, la forme pentamérique de celle-ci peut être réconstituée ; la protéine hybride conserve la capacité de CTB à se fixer aux récepteurs GM1 et présente l'épitope à sa surface sur une face n'interagissant pas avec le récepteur. Lorsque l'épitope est inséré au centre de la séquence de CTB, une faible proportion est reconstituée en pentamère ; la protéine hybride est peu ou pas capable de se fixer au récepteur et, s'il y a fixation, l'épitope ne peut pas être reconnu par un anticorps spécifique. Ces résultats sont en accord avec la prédiction de la structure tridimensionnelle de CTB.

L'invention concerne donc encore plus particulièrement.
1. Une méthode de production de vaccins contre toute infection intestinale qui utilise la protéine CTB comme adjuvant couplé par moyen de fusion de protéines a des protéines antigéniques.
2. Comme (1), mais où la fusion de l'antigène se fait par l'attachement de l'antigène à une des extrémités de la protéine CTB.
3. L'utilisation de protéines de fusion entre la protéine CTB comme adjuvant, et un antigène immunisation contre des infections intestinales.
4. Comme (3), mais où l'antigène est fusioné à une des extrémités de la protéine CTB.
5. Comme (4), mais où l'antigène est fusioné a la protéine CTB utilisant les gènes CTB modifiés CTBM2 et CTBM3.
6. Comme (4), mais où l'antigène est une protéine du virus gastoentérique du porc pour l'immunisation contre la maladie causée par ce virus.
7. Comme (6), mais où l'antigène est une protéine de la diarrhée bovine virale pour l'immunisation contre la maladie causée par ce virus.

### LEGENDE DES FIGURES

### Figure 1 :

Structure du plasmide pDL15 (5.000pb). Le fragment EcoRI-HindIII (600 pb) contient le gène LTB codant pour la sous-unité B de l'entérotoxine "Heat Labile" de E. coli.

### Figure 2 :

(a) Carte des sites de restriction conservés dans la région de DNA contenant le gène de la toxine cholérique selon Mekalanos et al., 1983.
(b) Carte de la région du gène CTB contenu dans le fragment XbaI-BglII isolé à partir de V cholera Ogawa..

- CTA :: DNA codant pour la sous-unité A de la toxine cholérique.
- CTB :: DNA codant pour la sous-unité B de la toxine cholérique.

### Figure 3 :

Clonage du gène CTB dans le plasmide pUC13 (2.730 pb) et structure du plasmide pUCT1 (5.000 pb). H et E : sites de restriction HincII et EcoRI.

### Figure 4(a) :

Clonage des fragments HincII-ClaI dans le vecteur M13tg131.
- NCTB :: fragment codant pour l'extrémité NH₂-terminale de CTB (223 pb).
- CCTB :: fragment codant pour l'extrémité COOH-terminale de CTB (146 pb).

### Figure 4(b) :

Clonage du fragment RsaI-ClaI dans le vecteur M13tg130.
- NCTB :: fragment codant pour l'extrémité NH₂-terminale de CTB (218 pb).

### Figure 5 :

La séquence du gène CTB cloné dans le plasmide pUCT1. La séquence des acides aminés de la protéine CTB est dérivée de la séquence nucléotidique. Les acides aminés de la protéine mature sont numérotés. La position des sites de restriction est indiquée par ^.

### Figure 6 :

Comparaison de la séquence de CTB de la souche 41 et d'autres souches. La séquence nucléotidique codant pour la sous-unité B mature de la toxine cholérique (souche classique 41) est représentée. Les acides aminés différents de la séquence de la souche 41 des sous-unités B des souches E1 Tor 2125 et E1 Tor 62746 sont représentées.

### Figure 7 :

Construction du plasmide pGMCTG contenant le gène CTB sous contrôle du promoteur PL du bactériophage Lambda.
- cI:: gène codant pour le répresseur thermosensible cI857.
- E, B, N et P :: sites de restriction EcoRI, BamHI, NdeI et PvuII. S-D : séquence Shine-Dalgarno.

### Figure 8 :

Schéma représentant la structure tridimensionnelle d'une sous-unité de la protéine capside du virus STNV. Les flèches représentent les brins bêta. Les lignes droites représentent les axes de symmétrie.

### Figure 9 :

Schéma représentant la structure tridimensionnelle prédite de la protéine CTB. Les flèches représentent le 8 brins bêta antiparallèles, et les nombres indiquent les résidus de la protéine situés respectivement au début et à la fin de chaque brin bêta. A5 représente l'axe de symmétrie du pentamère. La face de la protéine exposée au solvant et interagissant avec le récepteur GM1 est indiqué par la ligne pointillée.

### Figure 10 :

La structure du plasmide d'expression pSODL (3229 pb) portant le promoteur tac (ptac) et le gène hSOD codant pour la superoxide dismutase humaine. S-D : séquence Shine-Dalgarno.

### Figure 11 :

Schéma de la construction du plasmide pSODC, indiquant les séquences des oligonucléotides qui ont été insérés dans le plasmide pSODL.

### Figure 12 :

Schéma de la construction du plasmide pSCT9 portant le gène CTB-vecteur sous contrôle du promoteur ptac, après couplage cistronique au gène SOD.

### Figure 13 :

Schéma de structure des plasmides pAR3040 et pARAE, indiquant la séquence nucléotidique de la région d'insertion entre le promoteur du gène 10 (p10) et le terminateur du gène 10 (t10). SD indique la séquence Shine-Dalgarno du gène 10. Les sites de restriction BglII, PvuII, AvaII, PstI, EcoRI et EcoRV sont respectivement représentés par B, PII API, EI et EV.

### Figure 14 :

Schéma de la construction d'un vecteur intermédiaire pT7L (2.559 pb).
- p10 :: promoteur du gène de la protéine 10 du phage T7
- t10 :: terminateur du gène de la protéine 10 du phage T7
- S-D :: séquence Shine-Dalgarno.

### Figure 15 :

Schéma de la construction du plasmide pT7CTBR portant le gène CTBR sous contrôle du promoteur du gène 10 du bactériophage T7 (p10).
- t10 :: terminateur du gène de la protéine 10 du phage T7
- S-D :: séquence Shine-Dalgarno.

### Figure 16 :

Schéma de la structure du plasmide pT7CTBR2 portant le gène CTBR2 sous contrôle du promoteur du gène 10 du bactériophage T7.

### Figure 17 :

Analyse par PAGE-SDS 16% de l'expression des protéines CTBR (1) et CTBR2 (2) dans la souche BL21 (DE3).
- N et I :: Protéines bactériennes totales lorsque l'expression des protéines hétérologues n'est pas induite (N) ou est induite a l'IPTG (I).

Les bactéries contiennent respectivement les plasmides pT7CTBR (1) et pT7CTBR2 (2).

### Figure 18 :

Analyse par PAGE-SDS 16% des protéines CTB-vecteurs et des fusions CTB-antigènes présentes dans les corps d'inclusion purifiés des souches BL21 (DE3) portant les plasmides pCTBR (1), pCTBR2 (2), pCEPI3.1 (6), pCEPI3.2 (7), pCEPI3.3 (8), pCPC3.2 (9) et pCPC3.3 (10).

### Figure 19 :

Analyse par PAGE-SDS 16% des protéines CTB-vecteurs et des fusions CTB-antigènes purifiés des corps d'inclusion et rénaturées : CTBR (1), CTBR2 (2), CEPI3.1 (3), CEPI3.2 (4), CEPI3.3 (5), CPC3.2 (6) et CPC3.3 (7). P indique la position des pentamères; M indique la position des monomères.

### Figure 20 :

Schéma représentant le test GM1-ELISA. Les différentes étapes sont décrites dans le texte.
- A =: GM1
- B =: protéine à analyser
- C =: antisérum anti-CTB ou anticorps monoclonaux anti-antigènes couplés à la CTB
- D =: immunoglobulines de chèvre anti-immunoglobulines de lapin couplées à la péroxidase ou immunoglobulines de lapin anti-immunoglobulines de souris couplées à la péroxidase

### Figure 21 :

Comparaison de la réactivité des protéines CTBR et CTBR2 rénaturées par rapport à CTB Sigma^{(R)} dans le test GM1-ELISA : absorbance à 414 nm en fonction de la concentration en forme pentamérique des différentes protéines.
- A =: absorbance à 414nm
- C =: concentration en protéines

### Figure 22 :

Schéma de la construction des plasmides pCLHG1, pCLHG2 et pCLHG3, portant des fusions entre la protéine CTB-vecteur et le fragment F1 de la protéine E2 du virus de GET.

### Figure 23 :

Représentation schématisée de la structure des plasmides pCEPI3.1, pCEPI3.2 et pCEPI3.3 portant des fusions entre la protéine CTB-vecteur et l'épitope 3b5 du virus de la GET.
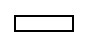 : DNA codant pour CTB
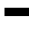 : DNA codant pour site C (épitope).

### Figure 24 :

Comparaison de la réactivité de la protéine CEPI3.1 par rapport à la protéine CTBR dans le test GM1-ELISA : absorbance à 414 nm en fonction de la concentration en forme pentamérique des différentes protéines.
- A =: absorbance à 414 nm
- C =: concentration en protéines.

### Figure 25 :

(a) Représentation schématisée de la structure des plasmides pCPC3.1, pCPC3.2 et pCPC3.3 portant des fusions entre la protéine CTB-vecteur et l'épitope C3 du virus de la poliomyélite.
   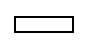 : ADN codant pour CTB
   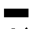 : ADN codant pour l'épitope 3C.
(b) Séquence de DNA codant pour l'épitope C3.

### Figure 26 :

Comparaison de la réactivité des protéines hybrides
(a) CEPI3.1 ; CEPI3.2 ; CEPI3.3 et
(b) CPC3.2 et CPC3.3
   par rapport à la protéine CTB Sigma -CTBS) dans le test GM1-ELISA : Absorbance à 414 nm en fonction de la concentration en forme pentamérique des différentes protéines.

- A :: Absorbance à 414 nm.

### Figure 27 :

Comparaison de la réactivité des protéines hybrides CEPI3.1, CEPI3.2, CEPI3.3 renaturées par rapport à CTBR dans le test GM1-ELISA anti-épitope du site C du virus de la GET.
- A =: voir fig. 26
- C =: voir fig. 26

### Figure 28 :

Comparaison de la réactivité des protéines hybrides CPC3.2 et CPC3.3 renaturées par rapport à CTBR dans le test GM1-ELISA anti-épitope C3 du virus de la poliomyélite.
- A =: voir fig. 26
- C =: voir fig. 26

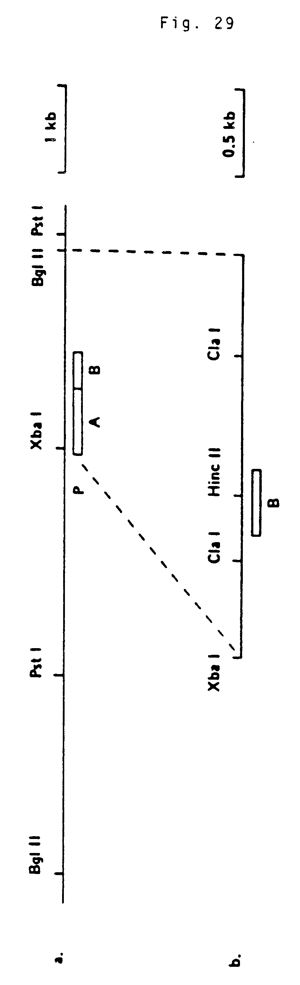

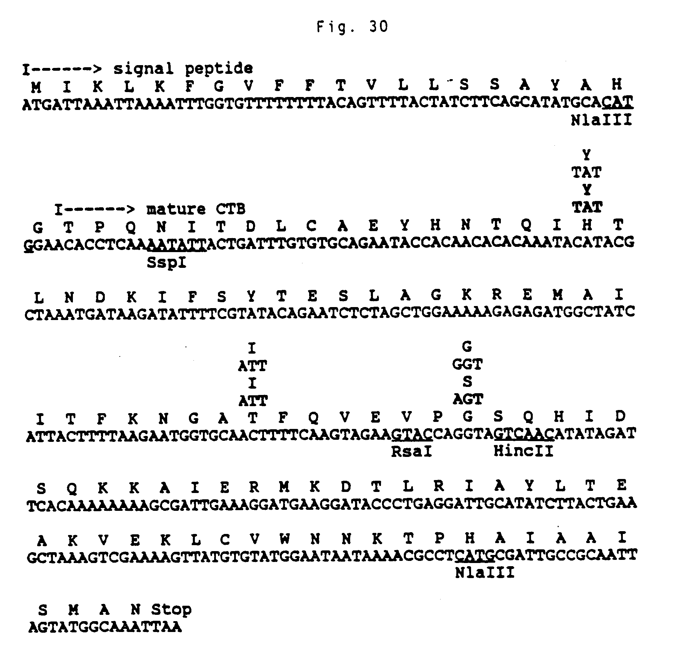

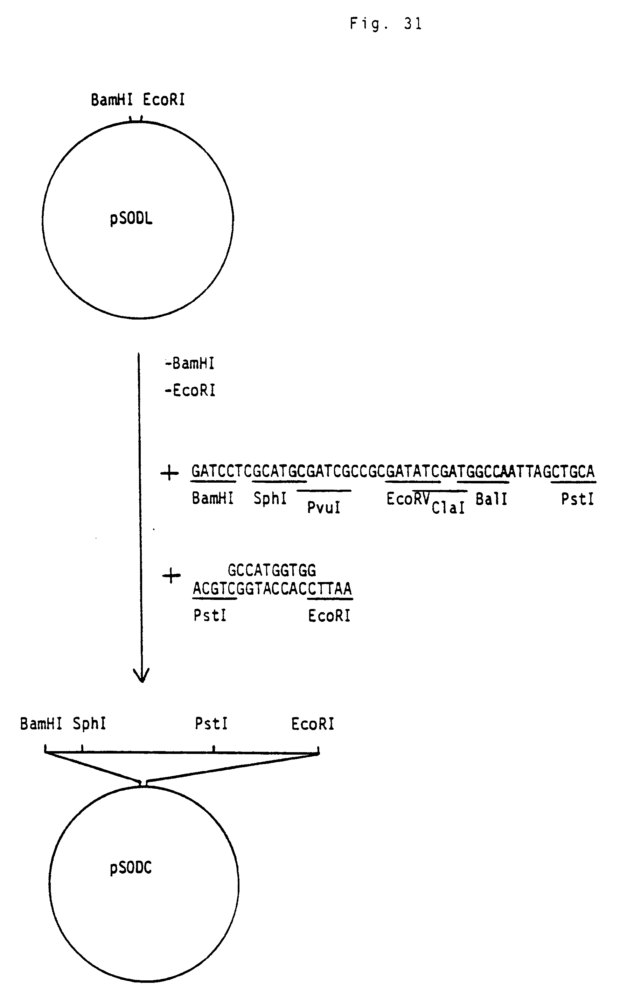

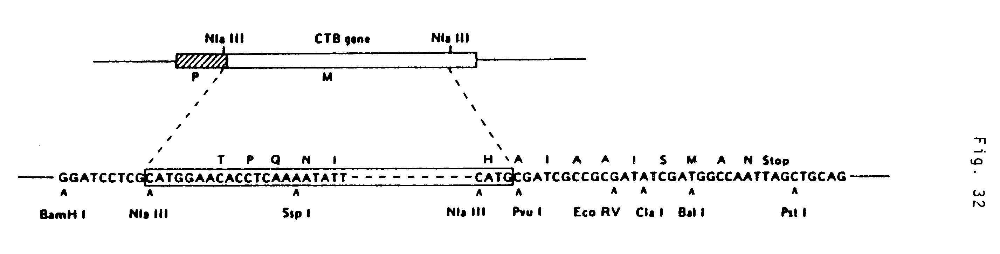

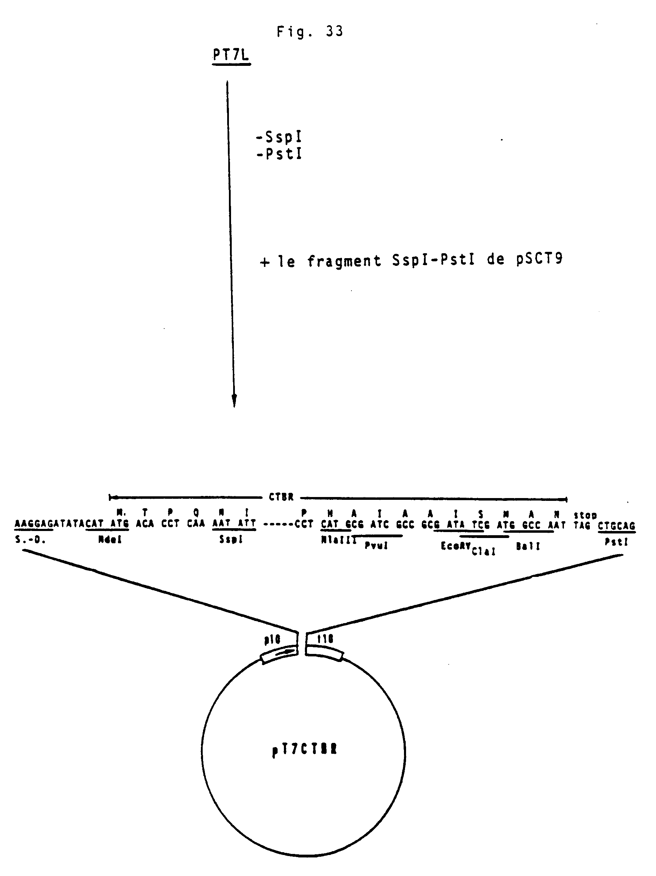

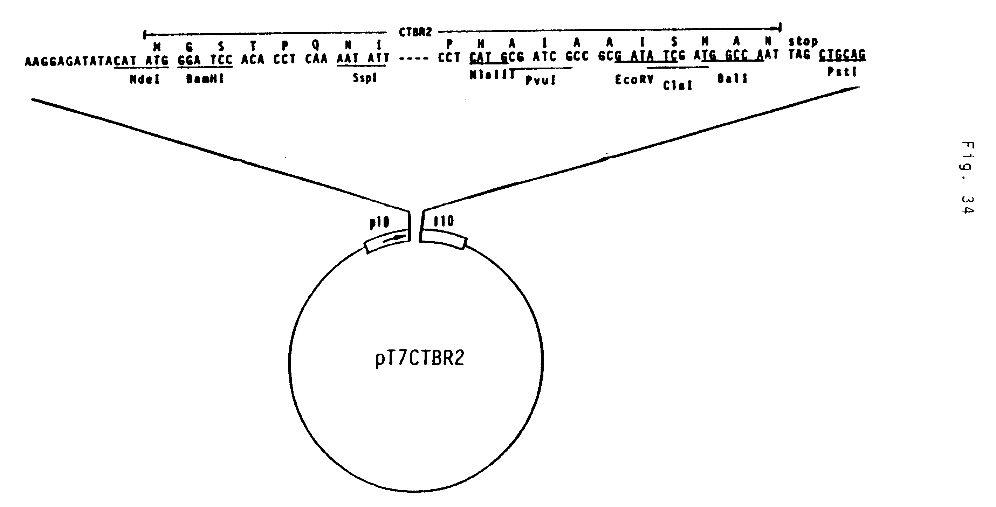

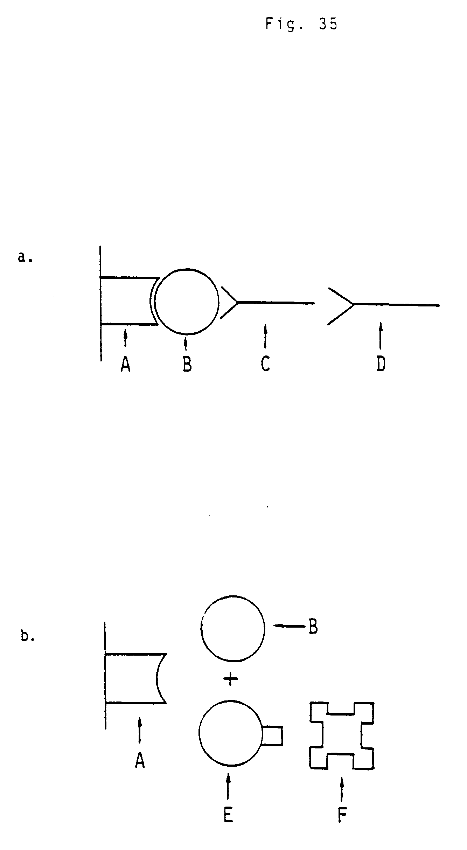

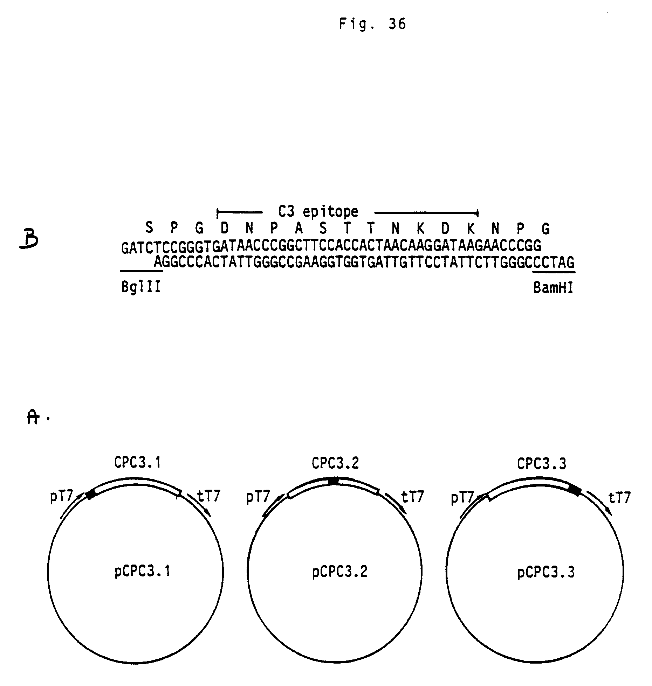

La description comprend également les figures 29, 30, 31, 32, 33, 34, 35 et 36 qui sont à rapprocher respectivement des figures 2, 5, 11, 12, 15, 16, 20 et 25.

### BIBLIOGRAPHIE

- -: Brenner D.J., Fanning G.R., Johnson K.E., Citarella R.V. and Falkow S. (1969). Polynucléotide Sequence Relationship among Members of Enterobacteriaceae. J. Bacteriol. 98 (2), 637-650.
- -: Bulletin de l'O.M.S. (1980). Immunité intestinale et élaboration de vaccins : mémorandum OMS. 58 (2), 252-274.
- -: Chen E.Y. and Seeburg P.H. (1985). Laboratory methods. Supercoil Sequencing : a fast and simple method for sequencing plasmid DNA. DNA. 4 (2), 165-170.
- -: Chou et Fasman. (1978). Adv. Enzymol. 47, 45-118.
- -: Cohen et al. (1986). J. Mol. Biol. 25, 266-275.
- -: De Boer H.A., Comstock L.J. and Vasser M. (1983). The tac promoter : a functional hybrid derived from the trp and lac promoters. Proc. Natl. Acad. Sci. USA. 80, 21-25.
- -: DeWolf M.J.S., Fridkin M., Epstein M. and Kohn, L.D. (1981). Structure-function studies of cholera toxin and its A and B promoters (modification fo trp residues). J. Biol. Chem. 256 (11), 5481-5488.
- -: DeWolf et al. (1981). J. Biol. Chem. 256, 5489-5496.
- -: Dallas W.S., Gill D.M. and Falkow S. (1979). Cistrons incoding Escherichia coli heat-labile toxin. J. Bacteriol. 139, 850-858.
- -: Divyer et Bloomfield. (1982). Biochem. 21, 3227-3231.
- -: Elson C.O. and Ealding W. (1984). Generalized systemic and mucosal immunity in mice after mucosal stimulation with cholera toxin. J. Immunol. 132 (6), 2736-2741.
- -: Eisenberg et al. (1984). J. Mol. Biol. 179, 125-142.
- -: Finkelstein R.A., Boesman M., Neoh S. H., LaRue M.K. and Delaney R. (1974). Dissociation and recombination of subunits of cholera enterotoxin (choleragen). J. Immunol. 113, 145-150.
- -: Fishman et al. (1978). Biochem. 17, 711-716.
- -: Garnier et al. (1986). J. Mol. Biol. 120, 97-120.
- -: Gennaro M.L., Greenaway P.J. and Broadbent D.A. (1982). The expression of biologically active cholera toxin in Escherichia coli. Nucl. Acid Res. 10 (16), 4883-4891.
- -: Gill D.M. (1976). The arrangement of subunits in cholera toxin. Biochem. 15 (6), 1242-1248.
- -: Hallewell R.A., Masiarz F.R., Najarian R. C., Puma J.P., Quiroga R., Randolph A., Sanchez-Pescador R., Scandella C.J., Smith B., Steimer K.S. and Mullenbach G.T. (1985). Human Cu/Zn superoxide dismutase cDNA : isolation of clones synthesising high levels of active or inactive enzyme from an expression library. Nucl. Acids Res. 13 (6), 2017-2034.
- -: Hirst T.R., Sanchez J., Kaper J.B., Hardy S.J.S. and Holmgren J. (1984). Mechanism of toxin secretion by Vibrio cholerae investigated in strains harboring plasmids that encode heat-labile enterotoxins of Escherichia coli. Proc. Natl. Acad. Sci. USA. 81, 7752-7756.
- -: Hirst T.R. and Holmgren J. (1987). Transient entry of enterotoxin subunits into the periplasm occurs during their secretion from Vibrio cholera. J. Bacteriol. 169 (3), 1037-1045.
- -: Hoghe et al. (1985). Science. 229, 1358-1365.
- -: Holmgren J., Lonnroth I., Mansson J.E. and Svennerholm L. (1975). Interaction of cholera toxin and membrane GM1 ganglioside of small intestine. Proc. Natl. Acad. Sci. USA. 72 (7), 2520-2524.
- -: Kurosky A., Markel D.E. and Peterson J.W. (1977). Covalent structure of the β-chain of cholera enterotoxin. J. Biol. Chem. 252, 7257-7264.
- -: Lai C.-Y. (1977). Determination of the primary structure of cholera toxin B subunit. J. Biol. Chem. 252, 7249-7256.
- -: Langley K.E., Berg T.F., Strickland T.W., Fenton D.M., Boone T.C. and Wypych J. (1987). Recombinant-DNA-derived bovine growth hormone from Escherichia coli. J. Biochem. 163, 313-321.
- -: Laude H. et Savey M. (1980). Les diarrhées néonatales d'origine virale chez le porc. Le Point Vétérinaire. 10, 83-89.
- -: Lockman H.A., Galen J.E. and Kaper J.B. (1984). Vibrio cholera enterotoxin genes : nucleotide sequence analysis of DNA encoding ADP-rybosyltransferase. J. Bacteriol. 159 (3), 1086-1089.
- -: Lockman H.A. and Kaper J.B. (1983). Nucleotide sequence analysis of the A2 and B subunits of Vibrio cholerae enterotoxin. J. Biol. Chem. 258 (22), 13722-13726.
- -: Lonroth I. and Holmgren J. (1975). Protein reagent modification of cholera toxin : characterization of effects on antigenic, receptor-binding and toxic properties. J. Gen. Microbiol. 91, 263-277.
- -: Lycke N. and Holmgren J. (1986). Strong adjuvant properties of cholera toxin on gut mucosal immune responses to orally presented antigens. Immunol. 59, 301-308.
- -: Lycke N. and Holmgren J. (1987). Long-term cholera antitoxin memory in the gut can be trigged to antibody formation associated with protection within hours of an oral challenge immunization. Scand. J. Immunol. 25 (4), 407-412.
- -: McKenzie S.J. and Halsey J.F. (1983). Mucosal immunization : a comparison of cholera toxin with 4 other proteins. Fed. Proc. 42, 863.
- -: McKenzie S.J. and Halsey J.F. (1984). Cholera toxin B subunit as a carrier protein to stimulate a mucosal immune response. J. Immunol. 133 (4), 1818-1824.
- -: Mekalanos J.J., Swartz D.J., Pearson G.D.N., Harford N., Groyne F. and de Wilde M. (1983a). Cholera toxin genes : nucleotide sequence, deletion analysis and vaccine development. Nature. 306, 551-557.
- -: Mekalanos J.J. (1983b). Duplication and amplification of toxin genes in Vibrio cholerae. Cell. 35, 253-263.
- -: Messing J. (1983). New M13 vectors for cloning. Methods enzymol. 101, 20-78.
- -: Moseley S.L. and Falkow S. (1980). Nucleotide sequence homology between the heat-labile enterotoxin gene of Escherichia coli and Vibrio cholerae deoxyribonucleic acid. J. Bacteriol. 144, 444-446.
- -: Nakashima Y., Napiorkowski P., Schafer D.E. and Koningsberg W.H. (1976). Primary structure of the B subunit of cholera enterotoxin. FEBS Lett. 68, 275-278.
- -: Pearson G.D.N. and Mekalanos J.J. (1982). Molecular cloning of Vibrio cholerae enterotoxin gene in E. coli K-12. Proc. Natl. Acad. Sci. USA. 79, 2976-2980.
- -: Pierce N.F. (1978). The role of antigen form and function in the primary and secondary intestinal immune responses to cholera toxin and toxoid in rats. J. Exp. Med. 148, 195-206.
- -: Ptisyn. (1981). FEBS Lett. 131, 197-202.
- -: Pugsley A.P. and Schwartz M. (1985). Export and secretion of proteins by bacteria. F.E.M.S. Microbiol. Rev. 32, 3-38.
- -: Rossman et al. (1983). J. Mol. Biol. 165, 711-732.
- -: Rossman et al. (1985). Nature. 317, 147-153.
- -: Sacks D.A., Huda S., Neogi P.K.B., Daniel R.R. and Spira W.M. (1980). Microtiter Ganglioside Enzyme-Linked ImmunoSorbent Assay for Vibrio and Escherichia coli heat-labile enterotoxins and antitoxin. J. Clin. Microbiol. 11 (1), 35-40.
- -: Schoner B.E., Hsiung H.M., Belagaje R.M. , Mayne N.G. and Schoner R.G. (1984). Role of mRNA translational efficiency in bovine growth hormone expression in Escherichia coli. Proc. Natl. Acad. Sci. USA. 81, 5403-5407.
- -: Sela et al. (1984). Modern approaches to vaccines, Chanock & Lerner eds., Cold Spring Harbor. 87-92.
- -: Siddel S., Wege H.,et Ter Meulen V. (1983). The Biology of Coronaviruses. J. Gen. Virol. 64, 761-776.
- -: Studier F.W. and Moffatt B.A. (1986). Use of bacteriophage T7 RNA polymerase to direct selective high-level expression of cloned genes. J. Mol. Biol. 189, 113-130.
- -: Sung W.L., Yao F.-L., Zahab D.M. and Narang S.A. (1986). Short synthetic oligodeoxyribonucleotide leader sequences enhance accumulation of human proinsulin synthesized in E. coli. Proc. Natl. Acad. Sci. USA. 83, 561-566.
- -: Tuggle C.K. and Fuchs J.A. (1985). Glutathione reductase is not required for maintenance of reduced glutathione in Escherichia coli K-12. J. Bacteriol. 162 (1), 448-450.
- -: Uren J. (1985). The recovery of genetically engineered mammalian proteins. Int. Biotechnol. Lab. 3 (1), 26-31.5.

## Revendications

1. Protéine hybride dérivée de la sous-unité B de la toxine cholérique et fusionnée avec une séquence active d'un antigène hétérologue vis-à-vis de ladite sous-unité et témoignant d'une activité Iga antigénique, cette protéine hybride conservant la spécificité d'attachement de la CTB aux gangliosides GM1 et possédant simultanément l'activité Iga antigénique de la protéine hétérologue,
caractérisée par le fait qu'elle comporte une séquence intacte de ladite sous-unité s'étendant entre le 3ème et le 100ème résidus d'acides aminés de la sous-unité B de la CTB et comptés à partir de l'extrémité N-terminale de cette sous-unité et que cette séquence est fusionnée en amont ou en aval de celle-ci avec la séquence active de l'antigène hétérologue.

2. Protéine hybride selon la revendication 1, caractérisée en ce que la protéine hétérologue comprend de 5 à 50 résidus d'acides aminés.

3. ADN recombinant codant pour la protéine hybride selon la revendication 1 ou la revendication 2.

4. Vecteur modifié par un insérat consistant en l'ADN recombinant selon la revendication 3, sous le contrôle d'un promoteur et des éléments de régulation appropriés permettant l'expression de cet ADN recombinant dans un hôte cellulaire compétent transformé par ledit vecteur recombinant.

5. Culture cellulaire transformée par le vecteur recombinant selon la revendication 4.

6. Culture cellulaire selon la revendication 5, caractérisée en ce que les cellules appartiennent à une espèce bactérienne apte à coloniser l'intestin des mammifères auxquels la protéine hybride doit être administrée.

## Claims

1. Hybrid protein derived from the cholera toxin B subunit and fused with an active sequence of an antigen heterologous in relation to the said subunit and exhibiting an antigenic IgA activity, this hybrid protein conserving the specificity of attachment of the CTB to the GM1 gangliosides and simultaneously possessing the antigenic IgA activity of the heterologous protein,
characterized by the fact that it contains an intact sequence of the said subunit stretching between the 3rd and the 100th amino acid residues of the B subunit of CTB and counted from the N-terminal end of this subunit, and that this sequence is fused upstream or downstream thereof with the active sequence of the heterologous antigen.

2. Hybrid protein according to Claim 1, characterized in that the heterologous protein comprises 5 to 50 amino acid residues.

3. Recombinant DNA encoding the hybrid protein according to Claim 1 or Claim 2.

4. Vector modified by an insert consisting of the recombinant DNA according to Claim 3, under the control of a promoter and appropriate regulatory elements permitting the expression of this recombinant DNA in a competent cellular host transformed by the said recombinant vector.

5. Cell culture transformed by the recombinant vector according to Claim 4.

6. Cell culture according to Claim 5, characterized in that the cells belong to a bacterial species capable of colonizing the intestine of the mammals to which the hybrid protein has to be administered.

## Patentansprüche

1. Hybrides Protein, das von der Untereinheit B des Choleratoxins abgeleitet und mit einer aktiven Sequenz eines hinsichtlich der genannten Untereinheit heterologen Antigens, das eine IgA-antigene Aktivität aufweist, fusioniert ist, wobei das hybride Protein die Spezifität hinsichtlich einer Anlagerung des CTB an Ganglioside GM1 bewahrt und gleichzeitig eine IgA-antigene Aktivität des heterologen Proteins besitzt,
dadurch gekennzeichnet,
daß es eine intakte Sequenz der genannten Untereinheit, die sich vom 3. bis zum 100. Aminosäurerest der Untereinheit B des CTB, gerechnet ausgehend vom N-terminalen Ende dieser Untereinheit, erstreckt, trägt und daß diese Sequenz stromaufwärts oder stromabwärts von ihr mit der aktiven Sequenz des heterologen Antigens fusioniert ist.

2. Hybrides Protein nach Anspruch 1,
dadurch gekennzeichnet, daß das heterologe Protein 5 bis 50 Aminosäurereste umfaßt.

3. Rekombinante DNS, die das hybride Protein nach Anspruch 1 oder 2 kodiert.

4. Vektor, modifiziert durch eine Insertionssequenz bestehend aus rekombinanter DNS nach Anspruch 3 unter der Kontrolle eines Promotors und geeigneter Regulationselemente, die die Expression dieser rekombinanten DNS in einem kompetenten zellulären Wirt, der mit dem rekombinanten Vektor transformiert worden ist, erlaubt.

5. Zellkultur, die mit dem rekombinanten Vektor nach Anspruch 4 transformiert worden ist.

6. Zellkultur nach Anspruch 5,
dadurch gekennzeichnet, daß die Zellen zu einer Bakteriengattung gehören, die geeignet ist, den Darm von Säugetieren, denen das hybride Protein verabreicht werden soll, zu besiedeln.
